# EUROPEAN PATENT APPLICATION

(11) **EP 4 372 007 A1**
(43) Date of publication of application: **22.05.2024**
(21) Application number: 22842513.8
(22) Date of filing: 15.07.2022
(51) Int. Cl.: C07K 16/28, A61P 27/02, A61P 43/00, A61K 39/00

(54) **HIF-1alpha INHIBITOR**

(30) Priority: 16.07.2021 KR 20210093191; 06.04.2022 KR 20220042695
(71) Applicant: NOVELTY NOBILITY Inc., Gyeonggi-do, 13477 (KR)
(72) Inventor: NA, Tae-Young, Seongnam-si Gyeonggi-do 13477 (KR)
(74) Representative: V.O.
(86) International application number: PCT/KR2022/010378
(87) International publication number: WO 2023/287251

(57) **Abstract**

The present disclosure relates to an inhibitor that blocks HIF-1α expression or activity. The inhibitor of the present disclosure effectively inhibits the expression of HIF-1α in the eye and downregulates the expression of angiogenic factors and/or inflammatory factors and, thus, can be usefully used for the prevention or treatment of eye disease.

## Description

### [Technical Field]

The present disclosure relates to a method for treating eye disease (e.g., retinal or macular disease), which includes a step of administering an inhibitor that blocks HIF-1α expression or activity.

### [Background Art]

HIF-1α (hypoxia-inducible factor 1-alpha) is a subunit of a hypoxia-inducible factor 1 (HIF-1) that is encoded by the HIF1A gene. HIF1A is considered as the master transcriptional regulator of cellular and developmental response to hypoxia (Wang GL et al., *Proceedings of the National Academy of Sciences of the United States of America.* 92 (12): 5510-4 (1995)). The dysregulation and overexpression of HIF1A by either hypoxia or genetic alterations have been heavily implicated in cancer biology, as well as a number of other pathophysiologies, specifically in areas of vascularization and angiogenesis, energy metabolism, cell survival and tumor invasion (Semenza GL, Nature Reviews. Cancer. 3 (10): 721-32 (2003)).

The retina is a transparent, thin layer located in the innermost part of the eye wall in contact with the vitreous body. It is the primary visual cortex that converts the optical information of objects into electrical signals and transmits images to the central visual system of the brain through the optic nerve. The retina consists of over 100 million light-sensing photoreceptor cells, over 1 million ganglion cells, which are visual neurons, and neurons that connect them. The macula is a nervous tissue located in the center of the retina. Most of visual cells are concentrated there and it is responsible for central vision because the images of objects are formed there. The macula lutea consists of a photoreceptor cell layer composed of cone cells and a ganglion cell layer. In bright light, the electrical signals of the images are converted to chemical signals and are transmitted to the brain via the optic nerve. The retinal region other than the macula lutea is responsible for peripheral vision and plays a major role when it is dark. Therefore, abnormality of the retina or macula due to aging or external factors can lead to visual impairment and blindness. Retinal or macular diseases include choroidal angiogenesis, macular degeneration, macular edema, retinopathy, retinal edema, retinal vascular occlusion, retinal detachment, glaucoma, etc.

The matters described above as background art are only for the purpose of improving the understanding of the background of the present disclosure and should not be taken as an admission that they are well known to those skilled in the art.

### [Disclosure]

### [Technical Problem]

The present disclosure is directed to providing a method for preventing or treating an eye disease that is refractory or non-responsive to existing eye disease drugs by inhibiting the expression or activity of HIF-1α, which includes a step of administering a therapeutically effective amount of an antibody or an antigen-binding fragment thereof, a nucleic acid molecule encoding the antibody or antigen-binding fragment thereof, or a vector containing the nucleic acid molecule to a subject in need thereof.

The present disclosure is also directed to providing a method for inhibiting the expression or activity of HIF-1α, which includes a step of administering a therapeutically effective amount of an antibody or an antigen-binding fragment thereof, a nucleic acid molecule encoding the antibody or antigen-binding fragment thereof, or a vector containing the nucleic acid molecule to a subject in need thereof.

The present disclosure is also directed to providing a method for preventing or treating an eye disease that is refractory or non-responsive to existing eye disease drugs, which includes a step of administering a therapeutically effective amount of an inhibitor that blocks HIF-1α expression or activity to a subject in need thereof.

The present disclosure is also directed to providing a pharmaceutical composition for preventing or treating an eye disease that is refractory or non-responsive to existing eye disease drugs, which contains an inhibitor that blocks HIF-1α expression or activity as an active ingredient.

Other purposes and advantages of the present disclosure will become more apparent by the following detailed description, claims and drawings.

### [Technical Solution]

The present disclosure relates to an inhibitor that blocks HIF-1α expression or activity and uses thereof. For example, eye disease, especially retinal or macular disease that is refractory or non-responsive to existing retinal or macular disease drugs, can be controlled, treated, alleviated, reduced or prevented from recurring by administration of an antibody inhibiting the expression or activity of HIF-1α or an antigen-binding fragment, a nucleic acid molecule encoding the same, or a vector containing the nucleic acid molecule.

A number of terms and phrases are defined to facilitate the understanding of the present disclosure. Additional definitions are presented in the detailed description.

### I. Definition

In the present specification, the term "about" is used to mean approximately, roughly, around or in the region of. When the term "about" is used in conjunction with a numerical range, it modifies the range by extending the boundaries above and below the presented numerical values. In general, the term "about" can modify a numerical value above and below, e.g., by 10%.

The terms "treat", "treating" and "treatment" used in the present specification refer to any kind of intervention or process performed on a subject or administration to a subject for the purpose of reversing, alleviating, improving, delaying or preventing the occurrence, progression, development, severity or recurrence of symptoms, complications, conditions or biological indicators related to a disease. The treatment may be performed on a subject having a disease or a subject not having a disease (e.g., for prevention).

The term "administration" used in the present specification refers to physically introducing a therapeutic agent or a composition containing a therapeutic agent to a subject using any of various methods and delivery systems known to those skilled in the art. The routes of administration of the antibody disclosed in the present specification include intravenous, intraperitoneal, intramuscular, subcutaneous, spinal or other parenteral routes of administration, e.g., injection or infusion. The term "parenteral administration" used in the present specification refers to modes of administration other than enteral and topical administration, typically by injection, including intravenous, intraperitoneal, intramuscular, intraarterial, intrathecal, intralymphatic, intralesional, intracapsular, intraorbital, intracardiac, intradermal, transtracheal, intratracheal, pulmonary, subcutaneous, subepidermal, intraocular, intraarticular, subtenon, subarachnoid, intracerebroventricular, intravitreal, epidural and substernal injection and infusion, as well as in-vivo electroporation, although not being limited thereto. The preferred route of administration of the composition described in the present specification includes intraocular administration.

In the present specification, the term "intraocular" means inside or under the eye tissue. The term "intraocular administration" used in the present specification refers to administration of a composition by sub-tenon, subconjunctival, suprachoroidal, subretinal, intravitreal or similar administrations. In some aspects, the intraocular administration includes suprachoroidal, subretinal and intravitreal administration.

In the present specification, the term "eye disease" refers to a disease occurring in the eye and includes an ocular vascular disease, a retinal or macular disease, a corneal disease, a conjunctival disease, a uveal disease, glaucoma, cataract, etc.

In the present specification, the terms "ocular vascular disease", "ocular vascular disorder" and "vascular eye disease" are used interchangeably and include, intraocular neovascular syndrome, e.g., diabetic retinopathy, diabetic macular edema, retinopathy of prematurity, neovascular glaucoma, retinal vein occlusion, central retinal vein occlusion, macular degeneration, age-related macular degeneration, retinitis pigmentosa, retinal angiomatous proliferation, macular telangectasia, ischemic retinopathy, iris neovascularization, intraocular neovascularization, corneal neovascularization, retinal neovascularization, choroidal neovascularization and retinal degeneration, although not being limited thereto (Garner, A., Vascular diseases, In: Pathobiology of ocular disease, A dynamic approach, Garner, A., and Klintworth, G.K., (eds.), 2nd edition, Marcel Dekker, New York (1994), pp. 1625-1710). As used herein, the ocular vascular disorder refers to any pathological condition characterized by altered or unregulated proliferation and invasion of new blood vessels into the structures of ocular tissues such as the retina and the cornea.

In an exemplary embodiment, the ocular vascular disease is selected from the group consisting of the following diseases: wet age-related macular degeneration (wet AMD), dry age-related macular degeneration (dry AMD), diabetic macular edema (DME), cystoid macular edema (CME), non-proliferative diabetic retinopathy (NPDR), proliferative diabetic retinopathy (PDR), vasculitis (e.g., central retinal vein occlusion), papilledema, retinitis, conjunctivitis, uveitis, choroiditis, multifocal choroiditis, ocular histoplasmosis, blepharitis, dry eye (Sjogren's disease) and other ophthalmic diseases (wherein the eye disease or disorder is associated with ocular neovascularization, vascular leakage and/or retinal edema). So, the antibody according to the present disclosure or an antigen-binding fragment thereof is useful for the prevention and treatment of wet AMD, dry AMD, CME, DME, NPDR, PDR, blepharitis, dry eye and uveitis, specifically wet AMD, dry AMD, blepharitis and dry eye, specifically CME, DME, NPDR and PDR, specifically blepharitis and dry eye, particularly wet AMD and dry AMD, particularly wet AMD. In some exemplary embodiments, the eye disease is selected from the group consisting of wet age-related macular degeneration (wet AMD), macular edema, retinal vein occlusion, retinopathy of prematurity and diabetic retinopathy.

The term "retinal disease" or "macular disease" used in the present specification refers to a disease, disorder or condition that affects or is associated with the choroid, retina or macula of the eye.

The retinal or macular disease that may be treated by the composition and method disclosed in the present specification includes one or more disease selected from the group consisting of choroidal angiogenesis, maculopathy, macular edema, retinopathy, retinal cell degeneration, retinal vascular occlusion, retinal detachment and glaucoma.

The term "retinopathy" used in the present specification refers to the disease or damage of the retina (i.e., the tissue lining the inner surface of the back of the eye that captures images passing through the cornea and lens) or retinal cells.

The term "diabetic retinopathy" (DR) used in the present specification refers to retinopathy caused by a complication associated with diabetes and includes all types of diabetic retinopathy such as non-proliferative diabetic retinopathy (NPDR), proliferative diabetic retinopathy (PDR), diabetic maculopathy and diabetic macular edema, although not being limited thereto.

Depending on the severity of the disease, DR may be asymptomatic, cause mild vision problems or lead to blindness. DR is the result of microvascular retinal changes. Hyperglycemia-induced intramural pericyte death and thickening of the basement membrane can lead to incompetence of the vascular walls. These damages change the formation of the blood-retinal barrier and make the retinal blood vessels become more permeable.

DR can be divided into two separate stages (Wu L., el al., World J Diabetes 4(6): 290-294 (2013)). The first stage, called non-proliferative diabetic retinopathy (NPDR), is associated with early diabetic retinopathy. NPDR generally has no apparent symptoms or is associated with minor vision distortion resulting from blood vessels leaking into the surrounding tissues. If left untreated, DR can progress into the second and more advanced stage, called proliferative diabetic retinopathy (PDR). PDR is characterized by abnormal new blood vessel formation (i.e., neovascularization), which can cause blurred vision through bursting and bleeding. Other symptoms of PDR include spots or dark threads ("floaters") floating in one's vision, change in vision, impaired color vision, dark or empty areas in one's vision, pain, patchy vision, complete vision loss, etc.

The term "maculopathy" used in the present specification refers to any pathological condition of the macula, an area at the center of the retina that is associated with highly sensitive, accurate vision. In some aspects, the terms "maculopathy" and "retinopathy" can be used interchangeably (when only the macula is affected). In some aspects, the maculopathy is diabetic maculopathy.

"Diabetic maculopathy" occurs when the macula is affected by the retinal changes caused by diabetes. The term refers to two distinct eye diseases: diabetic macular edema and diabetic ischemic maculopathy.

The term "macular degeneration" used in the present specification refers to any number of disorders and conditions in which the macula degenerates or loses functional activity. Age-related macular degeneration is the most common of the macular degeneration, but the term "macular degeneration" does not necessarily exclude macular degeneration in patients who are not the elderly. Non-limiting examples of macular degenerations include: age-related macular degeneration (wet or dry); Best's macular dystrophy, Sorsby's fundus dystrophy, mallatia leventinese, Doyne's honeycomb retinal dystrophy, Stargardt disease (also called Stargardt's macular dystrophy, juvenile macular degeneration, or fundus flavimaculatus), and pigment epithelial detachment-associated macular degeneration.

The term "age-related macular degeneration" (AMD) used in the present specification refers to retinopathy which usually affects the elderly and is associated with the loss of central vision, resulting from damage to the central part (i.e., macula) of the retina. AMD is generally characterized by progressive accumulation or aggregation of yellowish insoluble extracellular deposits, called drusen (buildup of extracellular proteins, such as amyloid beta, and lipids), in the macula (primarily between the retinal pigment epithelium (RPE) and the underlying choroid). The accumulation or aggregation of these deposits within the macula can cause gradual deterioration of the macula, resulting in central vision impairment. The term "macula" used in the present specification refers to the central part of the retina that is responsible for central, high-resolution, color vision.

There are two primary forms of AMD: (i) dry AMD and (ii) wet AMD. Unless specified otherwise, the term "age-related macular degeneration" includes both dry AMD and wet AMD. The term "age-related macular degeneration" used in the present specification also includes all types of age-related macular degeneration and all symptoms of age-related macular degeneration regardless of the cause. Non-limiting examples of the symptoms associated with macular degeneration (e.g., age-related macular degeneration) include loss of central vision, distortion, decreased contrast sensitivity, blurred vision, difficulty in adapting to low light level, sudden onset and rapid worsening of symptoms, and decreased color vision. In some aspects, macular degeneration (e.g., age-related macular degeneration) can cause macular edema (i.e., swelling of the macula resulting from collection of fluid and protein deposits on or under the macula).

The term "dry AMD" (also referred to as atrophic age-related macular degeneration or non-exudative AMD) refers to all forms of AMD that are not wet (neovascular) AMD. This includes early and intermediate forms of AMD, as well as the advanced form of dry AMD known as geographic atrophy. Dry AMD patients tend to have minimal symptoms in the earlier stages; visual function loss occurs more often if the condition advances to geographic atrophy.

The term "wet AMD" (also referred to as neovascular age-related macular degeneration or exudative AMD) used in the present specification refers to retinal condition characterized by the presence of retinal neovascularization and is the most advanced form of AMD. In wet AMD, blood vessels grow from the choriocapillaris through defects in Bruch's membrane and, in some cases, from the underlying retinal pigment epithelium (choroidal neovascularization or angiogenesis). The organization of serous or hemorrhagic exudates escaping from these vessels can result in fibrovascular scarring of the macular region with attendant degeneration of the neuroretina, detachment and tears of the retinal pigment epithelium, vitreous hemorrhage, and permanent loss of central vision.

The term "neovascularization" used in the present specification refers to the growth of new, abnormal blood vessels in different parts of the eyes that can result in bleeding and cause loss of vision. The term "choroidal neovascularization" used in the present specification refers to the abnormal growth of new blood vessels in the choroid (i.e., vascular layer of the eye, containing connective tissues and lying between the retina and the sclera). In wet AMD, the new blood vessels can grow through the retinal pigment epithelium (RPE) and choroid, and into the retina, and can cause damage to visual function due to blood and lipid leakage. The term "retinal neovascularization" used in the present specification refers to the abnormal development, proliferation and/or growth of blood vessels on or in the retina, e.g., on the retinal surface. The retinal neovascularization can develop in numerous retinopathies, such as retinopathies with retinal ischemia (e.g., diabetic retinopathy, sickle cell retinopathy, Eales disease, ocular ischemic syndrome, carotid cavernous fistula, familial exudative vitreoretinopathy, hyperviscosity syndrome, idiopathic occlusive arteriolitis, radiation retinopathy, retinal vein occlusion, retinal artery occlusion, retinal embolism, birdshot retinochoroidopathy, retinal vasculitis, sarcoidosis, toxoplasmosis, and uveitis, choroidal melanoma, chronic retinal detachment, anterior ischemic optic neuropathy (AION), non-arteritic anterior ischemic optic neuropathy (NAION) and incontinentia pigmenti).

The term "glaucoma" used in the present specification refers to a disease that causes damage of the optic nerve or optic neurons, leading to vision loss. Glaucoma is one of the main causes of blindness. Non-limiting examples of glaucoma include primary congenital glaucoma, open-angle glaucoma and closed-angle glaucoma.

The term "retinitis pigmentosa" (RP) used in the present specification refers to a genetic retinal disease that causes the damage (degeneration) of the optic cells and retinal pigment epithelial cells of the retina. As to the optic cells are damaged, nyctalopia appears in the early stage and the visual field narrows gradually, eventually leading to blindness.

The term "refractory" or "non-responsive" used in the present specification refers to a disease that does not respond to existing eye disease treatments or does not show enough effect for the existing treatments.

The term "existing eye disease drug" used in the present specification refers to an eye disease drug developed or marketed before the filing date of the present disclosure or a drug to which an eye disease treatment method with known mechanism of action has been applied.

The term "existing retinal or macular disease drug" used in the present specification may be a substance that inhibits the expression or activity of one or more target protein selected from the group consisting of VEGFA, VEGFB, PIGF, CCR3, VEGFR, PDGFR, CSF1R, FGF-2, PGF and Ang2. These substances include compounds, nucleic acids, peptides and viruses that can inhibit the expression or activity of the target proteins, vectors containing the nucleic acids, and siRNAs, antisense oligonucleotides, aptamers, antibodies or antigen-binding fragments thereof specific for the proteins, although not being limited thereto.

A specific example of the existing retinal or macular disease drug may be a VEGF inhibitor. The term "VEGF inhibitor" used in the present specification comprehensively refers to a substance that inhibits VEGF only or inhibits VEGF and other proteins (e.g., ANG-2) at the same time, and includes a substance in the form of the VEGF inhibitor conjugated with a biopolymer for increasing the duration of action, etc. Examples of the VEGF inhibitor include aflibercept (sold under the brand names Eylea^{®}, etc.), ranibizumab (sold under the brand names Lucentis^{®}, Susvimo^{®} Port Delivery System, etc.), bevacizumab (sold under the brand names Avastin^{®}, etc.), brolucizumab (sold under the brand names Beovu^{®}, etc.), pegaptanib (sold under the brand names Macugen^{®}, etc.), faricimab (sold under the brand names Vabysmo^{®}, etc.), KSI-301, and combinations thereof, although not being limited thereto.

The phrase "inhibit the expression or activity of HIF-1α " used in the present specification means any measurable decrease in the expression of the HIF1A gene or protein, or the activity of the HIF-1α protein, e.g., by at least about 10%, at least about 20%, at least about 30%, at least about 40%, at least about 50%, at least about 60%, at least about 70%, at least about 80%, at least about 90%, at least about 99%, or up to 100%.

The term "effective amount", "therapeutically effective amount" or "effective dose" is defined as an amount sufficient to achieve or at least partially achieve a desired effect. The "therapeutically effective amount" or "therapeutically effective dose" of a drug or a therapeutic agent refers to an amount that increases the frequency and duration of a period without disease symptoms evidenced by decrease in the severity of the symptoms or facilitates the prevention of injury or damage caused by the disease, when used alone or in combination with another therapeutic agent. The therapeutically effective amount or effective dose of a drug includes a "prophylactically effective amount" or a "prophylactically effective dose", which means the amount of the drug that inhibits the occurrence or recurrence of a disease when administered to a subject with a risk of developing the disease or suffering from the recurrence of the disease either alone or in combination with another therapeutic agent.

For example, for treatment of retinal or macular disease, the therapeutically effective amount or effective dose of the drug inhibits the expression or activity of HIF-1α by at least about 20%, at least about 40%, at least about 60%, or up to at least about 80% as compared to an untreated subject.

The term "antibody" is a term used in the art. It refers to a molecule with an antigen-binding site that binds specifically to an antigen. The term includes a whole antibody and any antigen-binding fragment (i.e., "antigen-binding region") or single chain thereof. In a specific exemplary embodiment, the "antibody" refers to a glycoprotein containing at least two heavy (H) chains and two light (L) chains interconnected by disulfide bonds, or an antigen-binding region thereof. In another specific exemplary embodiment, the "antibody" refers to a single-chain antibody containing a single variable domain, e.g., a VHH domain. Each heavy chain is composed of a heavy-chain variable region (abbreviated as V_{H}) and a heavy-chain constant region. In certain naturally-occurring antibodies, the heavy-chain constant region is comprised of three domains, C_{H}1, C_{H}2 and C_{H}3. In certain naturally-occurring antibodies, each light chain is composed of a light-chain variable region (abbreviated herein as V_{L}) and a light-chain constant region. The light-chain constant region is composed of one domain, C_{L}.

The V_{H} and V_{L} regions can be further subdivided into regions of hypervariability, termed complementarity-determining regions (CDRs), interspersed with regions that are more conserved, termed framework regions (FRs). Each V_{H} and V_{L} is composed of three CDRs and four FRs, arranged from the amino-terminus to the carboxy-terminus in the following order: FR1, CDR1, FR2, CDR2, FR3, CDR3 and FR4. The variable regions of the heavy and light chains contain a binding domain that interacts with an antigen. The constant regions of the antibodies can mediate the binding of the immunoglobulin to host tissues or factors, including various cells of the immune system (e.g., effector cells) and the first component of the classical complement system (C1q).

The antibody can be of any type (e.g., IgG, IgE, IgM, IgD, IgA or IgY), any class (e.g., IgD, IgG2, IgG3, IgG4, IgAl, or IgA2) or any subclass (e.g, lgG1, IgG2, IgG3, and IgG4 in humans; and lgG1, IgG2a, IgG2b, and IgG3 in mice) of immunoglobulin molecules. Immunoglobulins, e.g., IgG1, exist in several allotypes, which differ from each other in at most a few amino acids. The antibody disclosed in the present specification can be derived from any of the commonly known isotypes, classes, subclasses, or allotypes. In specific exemplary embodiments, the antibody disclosed in the present specification is of the IgG1, IgG2, IgG3, or IgG4 subclass, or any hybrid thereof. In specific exemplary embodiments, the antibody is of the human lgG1 subclass or the human IgG2 or human IgG4 subclass.

The "antibody" includes, for example, both naturally-occurring and non-naturally-occurring antibodies; monoclonal and polyclonal antibodies; chimeric and humanized antibodies; human and non-human antibodies; fully synthetic antibodies; single-chain antibodies; monospecific antibodies; multispecific antibodies (including bispecific antibodies); tetrameric antibodies containing two heavy chains and two light chains; antibody light-chain monomers; antibody heavy-chain monomers; antibody light-chain dimers; antibody heavy-chain dimers; antibody light chain-antibody heavy chain pairs; intrabodies; heteroconjugate antibodies; monovalent antibodies; camelized antibodies; affibodies; anti-idiotypic (anti-Id) antibodies (including, e.g., anti-anti-Id antibodies), and single-domain antibodies (sdAbs), which include binding molecules consisting of a single monomeric variable antibody domain that are fully capable of antigen binding (e.g., a V_{H} domain or a V_{L} domain) (Harmen M. M. and Haard H. J. Appl Microbiol Biotechnol. 77(1): 13-22 (2007)).

The term "antigen-binding region", i.e., "antigen-binding fragment" of an antibody used in the present specification refers to one or more fragment of an antibody that retains the ability to bind specifically to an antigen. Such "fragments" are, for example, between about 8 and about 1500 amino acids in length, suitably between about 8 and about 745 amino acids in length, suitably about 8 to about 300 amino acids, e.g., about 8 to about 200 amino acids, or about 10 to about 50 or 100 amino acids in length. It has been shown that the antigen-binding function of an antibody can be performed by fragments of a full-length antibody. Examples of binding fragments encompassed within the term "antigen-binding region" of an antibody include (i) a Fab fragment, a monovalent fragment consisting of the V_{L}, V_{H}, C_{L} and C_{H}1 domains; (ii) a F(ab')₂ fragment, a bivalent fragment containing two Fab fragments linked by a disulfide bridge at the hinge region; (iii) a Fd fragment consisting of V_{H} and C_{H}1 domains; (iv) a Fv fragment consisting of V_{L} and V_{H} domains of a single arm of an antibody, and disulfide-linked Fvs (sdFv); (v) a dAb fragment consisting of a V_{H} domain (Ward et al, (1989) Nature 341:544- 546); and (vi) an isolated complementarity-determining region (CDR) or (vii) a combination of two or more isolated CDRs which can optionally be joined by a synthetic linker, although not being limited thereto. Furthermore, although the two domains of the Fv fragment, V_{L} and V_{H}, are encoded for by separate genes, they can be joined using recombinant methods, by a synthetic linker that enables them to be made as a single protein chain in which the V_{L} and V_{H} regions pair to form monovalent molecules (known as single-chain Fv (scFv)) (see, e.g., Bird et al. (1988) Science 242: 423-426; and Huston et al. (1988) Proc. Natl. Acad. Sci. USA 85: 5879-5883). Such single-chain antibodies are also encompassed within the term "antigen-binding region" or "antigen-binding fragment" of an antibody. These antibody fragments are obtained using conventional techniques known to those skilled in the art, and the fragments are screened for utility in the same manner as that for intact antibodies. The antigen-binding regions can be produced by recombinant DNA techniques, or by enzymatic or chemical cleavage of intact immunoglobulins.

The term "variable region" or "variable domain" used in the present specification are used interchangeably in the art. The variable region typically refers to a portion of an antibody, generally, a portion of a light or heavy chain, typically about the amino-terminal 110 to 120 amino acids in the mature heavy chain and about 90 to 115 amino acids in the mature light chain, which differ extensively in sequence among antibodies and are used in the binding specificity of a particular antibody for its particular antigen. The variability in sequence is concentrated in those regions called complementarity-determining regions (CDRs), while the more highly conserved regions in the variable domain are called framework regions (FRs).

It is believed that the CDRs of the light and heavy chains are primarily responsible for the interaction and specificity of the antibody with an antigen. In specific exemplary embodiments, the variable region is a human variable region. In specific exemplary embodiments, the variable region includes rodent or murine CDRs and human framework regions (FRs). In specific exemplary embodiments, the variable region is a primate (e.g., non- human primate) variable region. In specific exemplary embodiments, the variable region includes rodent or murine CDRs and primate (e.g., non-human primate) framework regions (FRs).

The term "heavy chain" used in the present specification in reference to an antibody can refer to any distinct type, e.g., alpha (α), delta (δ), epsilon (ε), gamma (γ) and mu (µ), based on the amino acid sequence of the constant domain, which give rise to IgA, IgD, IgE, IgG and IgM classes of antibodies, respectively, including subclasses of IgG, e.g., IgG1, IgG2, IgG3 and IgG4.

The term "light chain" used in the present specification in reference to an antibody can refer to any distinct type, e.g., kappa (κ) or lambda (λ) based on the amino acid sequence of the constant domain. Light chain amino acid sequences are well known in the art. In specific exemplary embodiments, the light chain is a human light chain.

The terms "V_{L}" and "V_{L} domain" are used interchangeably to refer to the light-chain variable region of an antibody.

The terms "V_{H}" and "V_{H} domain" used interchangeably to refer to the heavy-chain variable region of an antibody.

The term "constant region" or "constant domain" used in the present specification are interchangeable and have meanings common in the art. The constant domain is a portion of an antibody, e.g., a carboxyl terminal portion of a light and/or heavy chain which is not directly involved in binding of an antibody to antigen but which can exhibit various effector functions, such as interaction with the Fc receptor. The constant region of an immunoglobulin molecule generally has a more conserved amino acid sequence relative to an immunoglobulin variable domain.

An "Fc region" (fragment crystallizable region), "Fc domain" or "Fc" refers to the C- terminal region of the heavy chain of an antibody that mediates the binding of the immunoglobulin to host tissues or factors, including binding to Fc receptors located on various cells of the immune system (e.g., effector cells) or to the first component of the classical complement system (C1q). Thus, an Fc region contains the constant region of an antibody excluding the first constant region immunoglobulin domain (e.g., C_{H}1 or C_{L}). In IgG, IgA and IgD antibody isotypes, the Fc region contains two identical protein fragments, derived from the second (C_{H}2) and third (C_{H}3) constant domains of the antibody's two heavy chains; IgM and IgE Fc regions contain three heavy-chain constant domains (C_{H} domains 2-4) in each polypeptide chain. For IgG, the Fc region contains immunoglobulin domains Cγ2 and Cγ3 and the hinge between Cγ1 and Cγ2. Although the boundaries of the Fc region of an immunoglobulin heavy chain might vary, the human IgG heavy chain Fc region is usually defined to stretch from an amino acid residue at position C226 or P230 (or amino acid between these two amino acids) to the carboxy-terminus of the heavy chain, wherein the numbering is according to the EU index as in Kabat. The C_{H}2 domain of a human IgG Fc region extends from about amino acid 231 to about amino acid 340, whereas the C_{H}3 domain is located on C-terminal side of a Cm domain in a Fc region, i.e., it extends from about amino acid 341 to about amino acid 447 of an IgG. The Fc region can be a native sequence Fc, including any allotypic variant, or a variant Fc (e.g., a non-naturally-occurring Fc). The Fc can also refer to an isolated region or in the context of an Fc-containing protein polypeptides such as a "binding protein containing an Fc region," also referred to as an "Fc fusion protein" (e.g., an antibody or immunoadhesion).

A "hinge", "hinge domain", "hinge region" or "antibody hinge region" refers to the domain of a heavy-chain constant region that joins the C_{H}1 domain to the C_{H}2 domain and includes the upper, middle, and lower portions of the hinge (Roux et al. J. Immunol. 1998 161: 4083). The hinge provides varying levels of flexibility between the binding and effector regions of an antibody and also provides sites for intermolecular disulfide bonding between the two heavy-chain constant regions. The sequences of wild-type IgG1, IgG2, IgG3 and IgG4 hinges are known in the art (see, e.g., Rabat EA et al., (1991) Sequences of Proteins of Immunological Interest, Fifth Edition, U.S. Department of Health and Human Services, NIH Publication No. 91-3242; Vidarsson G. et al. Front Immunol. 5:520 (published online on Oct. 20, 2014).

The term "isotype" used in the present specification refers to the antibody class (e.g., IgG1, IgG2, IgG3, IgG4, IgM, IgA1, IgA2, IgD and IgE antibodies) that is encoded by heavy-chain constant region genes.

The term "allotype" used in the present specification refers to naturally occurring varianst within a specific isotype group, which differ in a few amino acids (see, e.g., Jefferis et al. (2009) mAbs 1: 1). The antibodies described herein can be of any allotype. The allotypes of IgGl, IgG2, IgG3 and IgG4 are known in the art. (see, e.g., Kabat EA et al, (1991) supra; Vidarsson G. et al. Front Immunol. 5:520 (published online Oct. 20, 2014); and Lefranc MP, mAbs 1: 4, 1-7 (2009).

The term "isolated antibody" used in the present specification refers to an antibody which is substantially free of other antibodies having different antigen specificities.

The term "monoclonal antibody" used in the present specification refers to an antibody that displays a single binding specificity and affinity for a particular epitope, or a composition of antibodies in which all the antibodies display a single binding specificity and affinity for a particular epitope. Accordingly, the term "human monoclonal antibody" refers to an antibody or antibody composition that displays a single binding specificity and which has variable and optional constant regions derived from human germline immunoglobulin sequences. In a specific exemplary embodiment, human monoclonal antibodies are produced by a hybridoma which includes a B cell obtained from a transgenic non-human animal, e.g., a transgenic mouse, having a genome containing a human heavy-chain transgene and a light-chain transgene fused to an immortalized cell.

The term "recombinant human antibody" used in the present specification includes all human antibodies that are prepared, expressed, created or isolated by recombinant means, such as (a) antibodies isolated from an animal (e.g., mouse) that is transgenic or transchromosomal for human immunoglobulin genes or a hybridoma prepared therefrom, (b) antibodies isolated from a host cell transformed to express the antibody, e.g., from a transfectoma, (c) antibodies isolated from a recombinant, combinatorial human antibody library, and (d) antibodies prepared, expressed, created or isolated by any other means that involve splicing of human immunoglobulin gene sequences to other DNA sequences. Such recombinant human antibodies contain variable and constant regions that utilize particular human germline immunoglobulin sequences which are encoded by the germline genes but include subsequent rearrangements and mutations occurring, for example, during antibody maturation. As known in the art (see, e.g., Lonberg (2005) Nature Biotech. 23(9): 1117-1125), the variable region contains the antigen-binding domain, which is encoded by various genes that rearrange to form an antibody specific for a foreign antigen. In addition to rearrangement, the variable region can be further modified by multiple single amino acid changes (referred to as somatic mutation or hypermutation) to increase the affinity of the antibody to the foreign antigen. The constant region will change in further response to an antigen (i.e., isotype switch). Therefore, the rearranged and somatically mutated nucleic acid molecules that encode the light-chain and heavy-chain immunoglobulin polypeptides in response to an antigen cannot have sequence identity with the original nucleic acid molecules, but instead will be substantially identical or similar (i.e., at least 80% identity).

The term "human" antibody (HuMAb) used in the present specification refers to an antibody having variable regions in which both the framework and CDR regions are derived from human germline immunoglobulin sequences. Furthermore, if the antibody contains a constant region, the constant region is also derived from human germline immunoglobulin sequences. The antibodies described in the present specification can include amino acid residues not encoded by human germline immunoglobulin sequences (e.g., mutations introduced by random or site-specific mutagenesis in vitro or by somatic mutation in vivo). However, the term "human antibody" is not intended to include antibodies in which CDR sequences derived from the germline of another mammalian species, such as a mouse, have been grafted into human framework sequences. The terms "human" antibody and "fully human" antibody are used synonymously.

The term "humanized antibody" used in the present specification refers to an antibody in which some, most or all of the amino acids outside the CDR domain of a non-human antibody are replaced with corresponding amino acids derived from human immunoglobulins. In a specific exemplary embodiment of a humanized form of an antibody, some, most or all of the amino acids outside the CDR domain have been replaced with amino acids from human immunoglobulins, whereas some, most or all amino acids within one or more CDR region are unchanged. Additions, deletions, insertions, substitutions or modifications of amino acids are permissible as long as they do not abrogate the ability of the antibody to bind to a particular antigen. A "humanized" antibody retains an antigenic specificity similar to that of the original antibody.

The term "chimeric antibody" refers to an antibody in which the variable regions are derived from one species and the constant regions are derived from another species, such as an antibody in which the variable regions are derived from a mouse antibody and the constant regions are derived from a human antibody.

### II. Method for inhibiting expression or activity of HIF-1α

In an aspect, the present disclosure provides a method for inhibiting the expression or activity of HIF-1α, which includes a step of administering a therapeutically effective amount of the antibody described above or an antigen-binding fragment thereof, a nucleic acid molecule encoding the antibody or antigen-binding fragment thereof, or a vector containing the nucleic acid molecule to a subject in need thereof.

In an aspect, the present disclosure provides a method for preventing or treating an eye disease (e.g., retinal or macular disease) which is refractory or non-responsive to existing eye disease (e.g., retinal or macular disease) drugs by inhibiting the expression or activity of HIF-1α, which includes a step of administering a therapeutically effective amount of the antibody described above or an antigen-binding fragment thereof, a nucleic acid molecule encoding the antibody or antigen-binding fragment thereof, or a vector containing the nucleic acid molecule to a subject in need thereof.

In some specific exemplary embodiments, the antibody or antigen-binding fragment thereof includes the following CDR sequences:
(i) a light-chain CDR1 of the amino acid sequence represented by SEQ ID NO 1;
(ii) a light-chain CDR2 of the amino acid sequence represented by SEQ ID NO 2;
(iii) a light-chain CDR3 of the amino acid sequence represented by SEQ ID NO 3;
(iv) a heavy-chain CDR1 of the amino acid sequence represented by SEQ ID NO 4;
(v) a heavy-chain CDR2 of the amino acid sequence represented by SEQ ID NO 5; and
(vi) a heavy-chain CDR3 of the amino acid sequence represented by SEQ ID NO 6.

In some specific exemplary embodiments, the antibody or antigen-binding fragment thereof includes a light-chain variable region of the amino acid sequence represented by SEQ ID NO 7 and a heavy-chain variable region of the amino acid sequence represented by SEQ ID NO 8.

In some specific exemplary embodiments, the nucleic acid molecule encoding the antibody or antigen-binding fragment thereof may include base sequences of SEQ ID NO 9, SEQ ID NO 10 and SEQ ID NO 11. Each of the base sequences of SEQ ID NO 9, SEQ ID NO 10 and SEQ ID NO 11 respectively encodes the light-chain CDR1 represented by SEQ ID NO 1, the light-chain CDR2 represented by SEQ ID NO 2 and the light-chain CDR3 represented by SEQ ID NO 3. Each of the base sequences of SEQ ID NOS 9-11 may be codon-optimized for CHO cells. It is to be understood that nucleic acids including such codon-optimized base sequences are also encompassed within the scope of nucleic acids that encode the antibody according to the present disclosure or an antigen-binding fragment thereof. For example, a nucleic acid encoding the antibody according to the present disclosure or an antigen-binding fragment thereof may include SEQ ID NO 17, SEQ ID NO 18 and SEQ ID NO 19.

Optionally, the nucleic acid molecule encoding the antibody or antigen-binding fragment thereof may include base sequences of SEQ ID NO 12, SEQ ID NO 13 and SEQ ID NO 14. Each of the base sequences of SEQ ID NO 12, SEQ ID NO 13 and SEQ ID NO 14 respectively encodes the heavy-chain CDR1 represented by SEQ ID NO 4, the heavy-chain CDR2 represented by SEQ ID NO 5 and the heavy-chain CDR3 represented by SEQ ID NO 6. Each of the base sequences of SEQ IDS NO 12-14 may be codon-optimized for CHO cells. It is to be understood that nucleic acids including such codon-optimized base sequences are also encompassed within the scope of nucleic acids that encode the antibody according to the present disclosure or an antigen-binding fragment thereof. For example, a nucleic acid encoding the antibody according to the present disclosure or an antigen-binding fragment thereof may include SEQ ID NO 20, SEQ ID NO 21 and SEQ ID NO 22.

In an aspect of the present disclosure, a nucleic acid encoding the antibody according to the present disclosure or an antigen-binding fragment thereof may include a light-chain variable region-encoding nucleic acid including SEQ ID NO 15 or SEQ ID NO 23.

In another aspect of the present disclosure, a nucleic acid encoding the antibody according to the present disclosure or an antigen-binding fragment thereof may include a heavy-chain variable region-encoding nucleic acid including SEQ ID NO 16 or SEQ ID NO 24.

In another aspect of the present disclosure, the antibody according to the present disclosure or an antigen-binding fragment thereof may include a light chain including SEQ ID NO 25.

In another aspect of the present disclosure, the antibody according to the present disclosure or an antigen-binding fragment thereof may include a heavy chain including SEQ ID NO 26.

In another aspect of the present disclosure, a nucleic acid encoding the antibody or antigen-binding fragment thereof of the present disclosure may include a light chain-encoding nucleic acid including SEQ ID NO 27 or SEQ ID NO 29.

In another aspect of the present disclosure, a nucleic acid encoding the antibody or antigen-binding fragment thereof according to the present disclosure may include a heavy chain-encoding nucleic acid including SEQ ID NO 28 or SEQ ID NO 30.

In some specific exemplary embodiments, the antibody is the 2G4 antibody described in WO 2020/076105.

The antibody or antigen-binding fragment thereof (including an antibody formed as a nucleic acid molecule encoding the antibody or antigen-binding fragment thereof, or a vector containing the nucleic acid molecule being expressed) inhibits the expression or activity of HIF-1α, specifically in vivo, more specifically in the eye, further more specifically in the choroid or retina, although not being limited thereto.

The antibody or antigen-binding fragment thereof (including an antibody formed as a nucleic acid molecule encoding the antibody or antigen-binding fragment thereof, or a vector containing the nucleic acid molecule is expressed) inhibits the expression or activity of HIF-1α in cells in the eye, specifically the cells selected from the group consisting of optic neurons, retinal Mueller cell, choroidal cells, retinal microvascular endothelial cells (RMECs), retinal pigment epithelial (RPE) cells and photoreceptor cells, although not being limited thereto.

Examples of the diseases caused by the dysfunction or damage of choroidal cells, retinal microvascular endothelial cells (RMECs), retinal pigment epithelial (RPE) cells and photoreceptor cells include choroidal angiogenesis, maculopathy, retinopathy, retinal cell degeneration, retinal detachment, retinal vascular occlusion (PDR), etc. and the examples of the diseases caused by the dysfunction or damage of optic neurons and retinal Mueller cells include glaucoma, etc.

The inhibition of the expression of HIF-1α refers to the inhibition of the entire process including protein synthesis and regulation of protein degradation following the expression of the HIF-1α gene or a part thereof. It may refer specifically to the expression of mRNA or protein expression, more specifically to the inhibition of activity through facilitation of protein degradation.

The inhibition of the activity of HIF-1α may be achieved through the inhibition of the function of the HIF-1α protein produced from the expression, facilitated degradation of the HIF-1α protein, the inhibition of the activity of HIF-1α due to SCF-induced phosphorylation, etc.

In the present disclosure, the inhibition of the expression or activity of HIF-1α is not distinguished strictly and may be used interchangeably.

The administration of the antibody or antigen-binding fragment thereof (including an antibody formed as a nucleic acid molecule encoding the antibody or antigen-binding fragment thereof, or a vector containing the nucleic acid molecule being expressed, or an antigen-binding fragment thereof) inhibits the expression or activity of HIF-1α by at least 5%, at least 10%, at least 20%, at least 30%, at least 40%, at least 50%, at least 60%, at least 70%, at least 80%, at least 90% or up to 100% as compared to a subject to which it is not administered.

In some specific exemplary embodiments, the antibody or antigen-binding fragment thereof (including an antibody formed as a nucleic acid molecule encoding the antibody or antigen-binding fragment thereof, or a vector containing the nucleic acid molecule being expressed) further regulates angiogenic factors and/or inflammatory factors.

The angiogenic factors and/or inflammatory factors may be one or more factors selected from the group consisting of angiogenin, angiopoietin-2, CXCL16, endoglin, coagulation factor III, endotherin-1, MCP-1, EG-VEGF, VEGF, IGFBP-1, IGFBP-2, IL-1β, PDGF-AA, PIGF, serpin E, serpin F1, thrombospondin-2, ANGPT2, pentraxin 3, IL-8, FGF2 and FGF5.

In some specific exemplary embodiments, the antibody or antigen-binding fragment thereof (including an antibody formed as a nucleic acid molecule encoding the antibody or antigen-binding fragment thereof, or a vector containing the nucleic acid molecule being expressed) reduces the up-regulation of the expression of the angiogenic factors and/or inflammatory factors or down-regulates them.

The administration of the antibody or antigen-binding fragment thereof (including an antibody formed as a nucleic acid molecule encoding the antibody or antigen-binding fragment thereof, or a vector containing the nucleic acid molecule being expressed) reduces the up-regulation of the expression of the angiogenic factors and/or inflammatory factors by at least 5%, at least 10%, at least 20%, at least 30%, at least 40%, at least 50%, at least 60%, at least 70%, at least 80%, at least 90% or up to 100% as compared to a subject to which it is not administered.

In some specific exemplary embodiments, the antibody or antigen-binding fragment thereof (including an antibody formed as a nucleic acid molecule encoding the antibody or antigen-binding fragment thereof, or a vector containing the nucleic acid molecule being expressed) may reduce any one selected from a group consisting of the proliferation, migration and tube formation of microvascular cells.

### III. Method for preventing or treating eye disease

In another aspect of the present disclosure, the present disclosure provides a method for preventing or treating an eye disease, which includes a step of administering a therapeutically effective amount of an inhibitor that blocks HIF-1α expression or activity to a subject in need thereof.

In an aspect, the present disclosure provides a method for preventing or treating an eye disease that is refractory or non-responsive to existing eye disease (e.g., retinal or macular disease) drugs by inhibiting the expression or activity of HIF-1α, which includes a step of administering a therapeutically effective amount of the antibody or antigen-binding fragment thereof, a nucleic acid molecule encoding the antibody or antigen-binding fragment thereof, or a vector containing the nucleic acid molecule to a subject in need thereof.

In some specific exemplary embodiments, the inhibitor that blocks HIF-1α expression or activity includes an antibody including the following CDR sequences or an antigen-binding fragment thereof, a nucleic acid molecule encoding the antibody or antigen-binding fragment thereof, or a vector containing the nucleic acid molecule:
(i) a light-chain CDR1 of the amino acid sequence represented by SEQ ID NO 1;
(ii) a light-chain CDR2 of the amino acid sequence represented by SEQ ID NO 2;
(iii) a light-chain CDR3 of the amino acid sequence represented by SEQ ID NO 3;
(iv) a heavy-chain CDR1 of the amino acid sequence represented by SEQ ID NO 4;
(v) a heavy-chain CDR2 of the amino acid sequence represented by SEQ ID NO 5; and
(vi) a heavy-chain CDR3 of the amino acid sequence represented by SEQ ID NO 6.

In some specific exemplary embodiments, the antibody or antigen-binding fragment thereof includes a light-chain variable region of the amino acid sequence represented by SEQ ID NO 7 and a heavy-chain variable region of the amino acid sequence represented by SEQ ID NO 8.

In some specific exemplary embodiments, the nucleic acid molecule encoding the antibody or antigen-binding fragment thereof is the same as described above in <II. Method for inhibiting expression or activity of HIF-1α>.

In some specific exemplary embodiments, the antibody is the 2G4 antibody described in WO 2020/076105.

In some specific exemplary embodiments, the eye disease is one or more disease selected from the group consisting of an ocular vascular disease, a retinal or macular disease, a corneal disease, a conjunctival disease, a uveal disease, glaucoma and cataract.

In some specific exemplary embodiments, the retinal or macular disease is one or more disease selected from the group consisting of choroidal angiogenesis, maculopathy, retinopathy, retinal cell degeneration, retinal vascular occlusion, retinal detachment and glaucoma.

In some specific exemplary embodiments, the retinal or macular disease is one or more disease selected from the group consisting of diabetic retinopathy (DR), diabetic macular edema (DME), wet age-related macular degeneration (AMD), retinal vein occlusion (RVO), retinopathy of prematurity and glaucoma.

In some specific exemplary embodiments, the method disclosed in the present specification increases the possibility of treatment or reduces the treatment period (e.g., DR, DME, AMD, etc.) in a subject. For example, the treatment period is reduced by at least about 1 month, at least about 2 months, at least about 3 months, at least about 4 months, at least about 5 months, at least about 6 months, at least about 7 months, at least about 8 months, at least about 9 months, at least about 10 months, at least about 11 months, at least about 1 year or longer, as compared to a subject not treated with the antibody or composition disclosed in the present specification. In another specific exemplary embodiment, the method disclosed in the present specification increases the duration of the disease-weakened state in a subject by about 1 month or longer, about 2 months or longer, about 3 months or longer, about 4 months or longer, about 5 months or longer, about 6 months or longer, about 7 months or longer, about 8 months or longer, about 9 months or longer, about 10 months or longer, about 11 months or longer or about 1 year or longer, as compared to a subject not treated with the antibody or composition disclosed in the present specification.

In some specific exemplary embodiments, the method of the present disclosure increases the duration of the state where the progress of a disease in a subject is non-existent or delayed. For example, the duration of the state where the progress of a disease in a subject is non-existent or delayed is increased by at least about 1 month, at least about 2 months, at least about 3 months, at least about 4 months, at least about 5 months, at least about 6 months, at least about 7 months, at least about 8 months, at least about 9 months, at least about 10 months, at least about 11 months or at least about 1 year, for example, as compared to a subject not treated with the antibody or composition disclosed in the present specification.

In some specific exemplary embodiments, the method disclosed in the present specification effectively increases response rate in a group of patients. For example, the response rate in a group of patients is increased by at least about 2%, at least about 3%, at least about 4%, at least about 5%, at least about 10%, at least about 15%, at least about 20%, at least about 25%, at least about 30%, at least about 35%, at least about 40%, at least about 45%, at least about 50%, at least about 55%, at least about 60%, at least about 70%, at least about 75%, at least about 80%, at least about 85%, at least about 90%, at least about 95%, at least about 99% or at least about 100%, for example, as compared to a subject not treated with the antibody or composition disclosed in the present specification.

In some specific exemplary embodiments, the term "reference" refers to a corresponding subject (e.g., a subject having diabetic retinopathy or age-related macular degeneration) who does not receive a composition disclosed herein (e.g., an inhibitor that blocks HIF-1α expression or activity, e.g., an antibody). The term "reference" can also refer to the same subject (e.g., a subject having diabetic retinopathy or age-related macular degeneration) but prior to the administration of a composition disclosed in the present specification. In certain aspects, the term "reference" refers to an average population of subjects (e.g., subjects having diabetic retinopathy or age-related macular degeneration).

In some specific exemplary embodiments, the retinopathy includes diabetic retinopathy. In certain aspects, the diabetic retinopathy is non-proliferative diabetic retinopathy (NPDR). In other aspects, the diabetic retinopathy is proliferative diabetic retinopathy (PDR). In some aspects, the diabetic retinopathy is diabetic maculopathy. In other aspects, the diabetic retinopathy is diabetic macular edema. In some aspects, the diabetic retinopathy is any retinopathy associated with the ischemic damage within the retina.

In some specific exemplary embodiments, the macular degeneration that can be treated in the present disclosure includes age-related macular degeneration (AMD). In some aspects, the age-related macular degeneration is early AMD. In other aspects, the age-related macular degeneration is intermediate AMD. In further aspects, the age-related macular degeneration is late or advanced AMD (i.e., geographic atrophy). In some aspects, the age-related macular degeneration is dry (non-exudative) AMD. In other aspects, the age-related macular degeneration is wet (neovascular or exudative) AMD.

In some specific exemplary embodiments, the subject that can be treated with the method of the present disclosure is a nonhuman animal, such as rat or mouse. In other aspects, the subject that can be treated with the method of the present disclosure is human.

In an aspect, the "existing retinal or macular disease drug" in the method of the present disclosure may be a VEGF inhibitor, specifically one selected from the group consisting of aflibercept, ranibizumab, Susvimo^{®}, bevacizumab, brolucizumab, pegaptanib, faricimab and KSI-301.

### IV. Pharmaceutical composition or use

In another aspect, the present disclosure provides a pharmaceutical composition for preventing or treating an eye disease (e.g., a retinal or macular disease), which contains an inhibitor that blocks HIF-1α expression or activity as an active ingredient.

In some specific exemplary embodiments, the inhibitor that blocks HIF-1α expression or activity includes an antibody including the following CDR sequences or an antigen-binding fragment thereof, a nucleic acid molecule encoding the antibody or antigen-binding fragment thereof, or a vector containing the nucleic acid molecule:
(i) a light-chain CDR1 of the amino acid sequence represented by SEQ ID NO 1;
(ii) a light-chain CDR2 of the amino acid sequence represented by SEQ ID NO 2;
(iii) a light-chain CDR3 of the amino acid sequence represented by SEQ ID NO 3;
(iv) a heavy-chain CDR1 of the amino acid sequence represented by SEQ ID NO 4;
(v) a heavy-chain CDR2 of the amino acid sequence represented by SEQ ID NO 5; and
(vi) a heavy-chain CDR3 of the amino acid sequence represented by SEQ ID NO 6.

In another aspect of the present disclosure, the present disclosure provides a pharmaceutical composition for preventing or treating an eye disease (e.g., a retinal or macular disease) which is refractory or non-responsive to existing eye disease (e.g., retinal or macular disease) drugs by inhibiting the expression or activity of HIF-1α, which contains an antibody including the following CDR sequences or an antigen-binding fragment thereof, a nucleic acid molecule encoding the antibody or antigen-binding fragment thereof, or a vector containing the nucleic acid molecule as an active ingredient:
(i) a light-chain CDR1 of the amino acid sequence represented by SEQ ID NO 1;
(ii) a light-chain CDR2 of the amino acid sequence represented by SEQ ID NO 2;
(iii) a light-chain CDR3 of the amino acid sequence represented by SEQ ID NO 3;
(iv) a heavy-chain CDR1 of the amino acid sequence represented by SEQ ID NO 4;
(v) a heavy-chain CDR2 of the amino acid sequence represented by SEQ ID NO 5; and
(vi) a heavy-chain CDR3 of the amino acid sequence represented by SEQ ID NO 6.

In another aspect of the present disclosure, the present disclosure provides the use of antibody including the following CDR sequences or an antigen-binding fragment thereof, a nucleic acid molecule encoding the antibody or antigen-binding fragment thereof, or a vector containing the nucleic acid molecule, for prevention or treatment of an eye disease (e.g., a retinal or macular disease) which is refractory or non-responsive to existing eye disease (e.g., retinal or macular disease) drugs by inhibiting the expression or activity of HIF-1α:
(i) a light-chain CDR1 of the amino acid sequence represented by SEQ ID NO 1;
(ii) a light-chain CDR2 of the amino acid sequence represented by SEQ ID NO 2;
(iii) a light-chain CDR3 of the amino acid sequence represented by SEQ ID NO 3;
(iv) a heavy-chain CDR1 of the amino acid sequence represented by SEQ ID NO 4;
(v) a heavy-chain CDR2 of the amino acid sequence represented by SEQ ID NO 5; and
(vi) a heavy-chain CDR3 of the amino acid sequence represented by SEQ ID NO 6.

In some specific exemplary embodiments, the antibody or antigen-binding fragment thereof includes a light-chain variable region of the amino acid sequence represented by SEQ ID NO 7 and a heavy-chain variable region of the amino acid sequence represented by SEQ ID NO 8.

In some specific exemplary embodiments, the nucleic acid molecule encoding the antibody or antigen-binding fragment thereof is the same as described above in <II. Method for inhibiting expression or activity of HIF-1α>.

In some specific exemplary embodiments, the antibody is the 2G4 antibody described in WO 2020/076105.

In some specific exemplary embodiments, the eye disease is one or more disease selected from the group consisting of an ocular vascular disease, a retinal or macular disease, a corneal disease, a conjunctival disease, a uveal disease, glaucoma and cataract.

In some specific exemplary embodiments, the retinal or macular disease is one or more disease selected from the group consisting of choroidal angiogenesis, maculopathy, retinopathy, retinal cell degeneration, retinal vascular occlusion, retinal detachment and glaucoma.

In some specific exemplary embodiments, the retinal or macular disease is one or more disease selected from the group consisting of diabetic retinopathy, diabetic macular edema, wet macular degeneration, retinal vein occlusion, retinopathy of prematurity and glaucoma.

Compositions containing the antibody disclosed in the present specification having a desired degree of purity in a physiologically acceptable carrier, excipient or stabilizer (Remington's Pharmaceutical Sciences (1990) Mack Publishing Co., Easton, PA) are described in the present specification.

In some specific exemplary embodiments, the pharmaceutical composition contains the antibody or antigen-binding region, bispecific molecule or immunoconjugate described in the present specification in a pharmaceutically acceptable carrier. In some aspects, the pharmaceutical composition contains an effective amount of the antibody or antigen-binding portion thereof described in the present specification and, optionally, one or more additional prophylactic of therapeutic agent, in a pharmaceutically acceptable carrier. In some aspects, the antibody is the only active ingredient contained in the pharmaceutical composition. The pharmaceutical compositions described in the present specification can be useful in reducing the expression or activity of HIF-1α and, thus, in treating an eye disease, disorder or condition, such as AMD and glaucoma.

The pharmaceutical composition described in the present specification can be formulated for any administration route for a subject. In some aspects, the pharmaceutical composition described in the present specification may be administered via any route through which the composition can be delivered to sub-Tenon, subconjunctival, suprachoroidal, subretinal, intravitreal and like locations of the eye. In certain aspects, the pharmaceutical composition described in the present specification is delivered to a subject via intraocular administration. In some aspects, the intraocular administration includes intravitreal administration. In the present specification, parenteral administration by subcutaneous, intramuscular, intraperitoneal or intravenous injection is also considered.

The antibody or antigen-binding region thereof described in the present specification can be formulated as a gel, cream or lotion for topical application to skin and mucous membranes, such as in the eye, or for intracisternal or intrathecal application. Topical administration is contemplated for transdermal delivery and also for administration to the eye or mucosa or for inhalation therapy. A nasal solution of the antibody can be administered either alone or in combination with another pharmaceutically acceptable excipient.

In some aspects, the pharmaceutical composition containing the antibody or antigen-binding region thereof described in the present specification is a lyophilized powder which can be reconstituted for administration as a solution, emulsion or other mixtures. The lyophilized powder can be reconstituted and formulated as a solid or a gel.

The present disclosure provides a formulation for parenteral administration by reconstituting the lyophilized powder with water for injection. For the reconstitution, the lyophilized powder is added to sterile water or other suitable carriers. The precise amount depends on the selected compound and can be determined empirically.

### [Advantageous Effects]

The features and advantages of the present disclosure may be summarized as follows:
(i) The present disclosure provides inhibitors that block HIF-1α expression or activity.
(ii) The inhibitor that blocks HIF-1α expression or activity of the present disclosure effectively inhibits the expression of HIF-1α in the eye and downregulates the expression of angiogenic factors and/or inflammatory factors and, thus, can be usefully used for the prevention or treatment of eye disease.

### [Brief Description of Drawings]

FIG. 1 shows that NN2101 effectively suppresses the choroidal expression of HIF-1α in laser-induced CNV mice.
FIGS. 2a-2c show that NN2101 effectively suppresses the expression of HIF-1α in hypoxic state. FIG. 2a: protein levels in total cell lysate. FIGS. 2b and 2c: sSCF and sVEGFA secreted to culture supernatant.
FIGS. 3a-3b show that NN2101 regulates the expression of HIF-1α by SCF-induced phosphorylation. FIG. 3a: protein levels in total cell lysate. FIG. 3b: sSCF (top) and sVEGFA (bottom) secreted to culture supernatant.
FIG. 4 shows that NN2101 significantly reduces the expression of HIF-1α, VEGFA, mSCF and phospho-cKIT proteins in hypoxic state.
FIGS. 5a-5b show that the treatment of HRMECs with NN2101 regulates the mRNA levels of the HIF-1α target gene in hypoxic state. FIG. 5a: induction of hypoxic state by treating with CoCl₂. FIG. 5b: verification using hypoxic chamber.
FIGS. 6a-6b show that the treatment of RPE cells with NN2101 regulates the mRNA levels and protein levels of the HIF-1α target gene in hypoxic state. FIG. 6a: protein levels. FIG. 6b: mRNA levels.
FIG. 7 shows a result of analyzing the protein expression level of a cell lysate by western blotting after treating HRMEC, ARPE19, MIO-M1 and Y79 cells with NN2101 and then inducing hypoxic state (1% O₂).
FIGS. 8a-8b and 9 show that NN2101 facilitates the degradation of HIF-1α via the ubiquitin-proteasome degradation pathway. FIG. 8a: treatment with MG132. FIG. 8b: treatment with CHx. FIG. 9: HIF-1α-PHD analysis result.
FIGS. 10-13 show that NN2101 regulates neovascularization/inflammatory factors in HRMEC, ARPE19, MIO-M1 and Y79 cells. FIG. 10: HRMEC cells. FIG. 11: ARPE19 cell. FIG. 12: MIO-M1 cells. FIG. 13: Y79 cells.

### [Best Mode]

Hereinafter, the present disclosure will be described in more detail through examples. The examples are solely for describing the present disclosure more specifically and it will be obvious to those having ordinary knowledge in the art that the scope of the present disclosure is not limited by the examples.

### Examples

### Example 1. Screening of NN2101 antibody as HIF-1α inhibitor

Antibodies exhibiting activity as HIF-1α inhibitors (inhibitors that block HIF-1α expression or activity) were screened using an antibody pool of Novelty Nobility.

The CDR sequences of the screened NN2101 antibodies are given in Table 1.

**[Table 1]**

| CDR | Sequence | SEQ ID NO |
|---|---|---|
| L-CDR1 | QSLLHSNGYN Y | SEQ ID NO: 1 |
| L-CDR2 | LGS | SEQ ID NO: 2 |
| L-CDR3 | MQALQTIT | SEQ ID NO: 3 |
| H-CDR1 | GFTFSRYG | SEQ ID NO: 4 |
| H-CDR2 | IWYDGTNK | SEQ ID NO: 5 |
| H-CDR3 | AREDWAEAFD M | SEQ ID NO: 6 |

The sequences of the light-chain variable regions and heavy-chain variable regions of the NN2101 antibodies are shown in Table 2.

**[Table 2]**

| Variable region | Sequence | SEQ ID NO |
|---|---|---|
| Light chain | DIVMTQSPLS LPVTPGEPAS ISCRSSQSLL HSNGYNYLDW YLQKPGQSPQ LLIYLGSNRA SGVPDRFSGS GSGTDFTLKI SRVEAEDVGV YYCMQALQTI TFGQGTRLEI K | SEQ ID NO: 7 |
| Heavy chain | QVQLVESGGG VVQPGRSLRL SCAASGFTFS RYGMHWVRQA PGKGLEWVAV IWYDGTNKDY TDSVRGRFTI SRDNSKNTLY LQMNSLRAED TAVYYCARED WAEAFDMWGQ GTTVTVSS | SEQ ID NO: 8 |

### Example 2. NN2101 down-regulates HIF-1α expression in murine CNV model.

A laser-induced CNV mouse model was established as follows. Choroidal neovascularization was induced by damaging the Bruch's membrane of one eye of a 10-week-old C57BL/6 mouse (Orient Co., Ltd., Korea). The used laser was as follows: laser photocoagulation: wavelength: 532 nm; fixed diameter: 50 µm; intensity: 300 mW; duration: 0.70.

Immediately after the laser photocoagulation, NN2101 (1 µg/1 µL) was administered into one eye of the CNV mouse by intravitreal injection. PBS (1 µL) was administered intravitreally into the other eye served as a control. On day 7 after the laser photocoagulation, the eyes were extracted from the laser-induced CNV mouse. Pho-cKIT, pho-GSK-3β, HIF-1α and VEGF-A proteins in the choroidal tissue extracted therefrom were quantified by conducting western blot (hereinafter, also referred to as "WB").

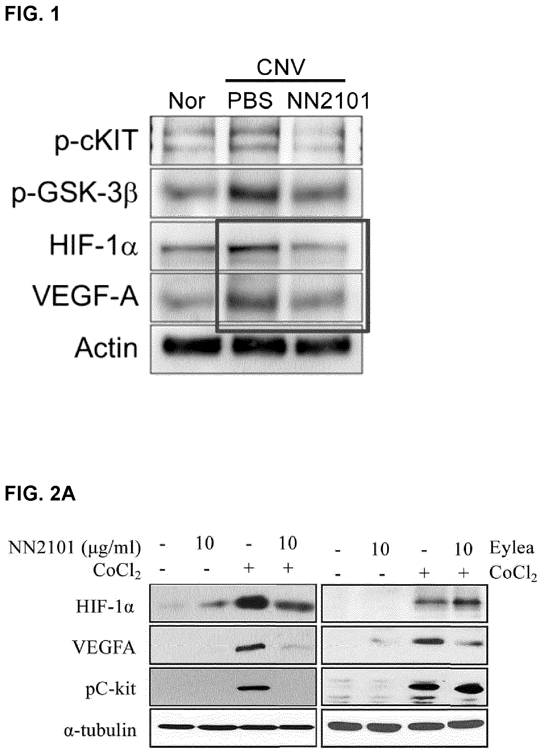

The intravitreal injection of NN2101 suppressed the choroidal expression of HIF-1α in the CNV mouse and the expression of VEGF-A and pho-cKIT was also decreased (FIG. 1).

### Example 3: NN2101 completely inhibits HIF-1α in hypoxic HRMECs (human retinal microvascular endothelial cells) unlike aflibercept.

HIF-1α is expressed in hypoxic state. HIF-1α also stimulates the expression of angiogenic factors including SCF, VEGF, etc. and, as a result, SCF/cKIT and VEGF signaling mechanisms are activated, respectively. In this example, it was investigated whether NN2101 inhibits the expression of HIF-1α in CoCl₂-induced hypoxic state.

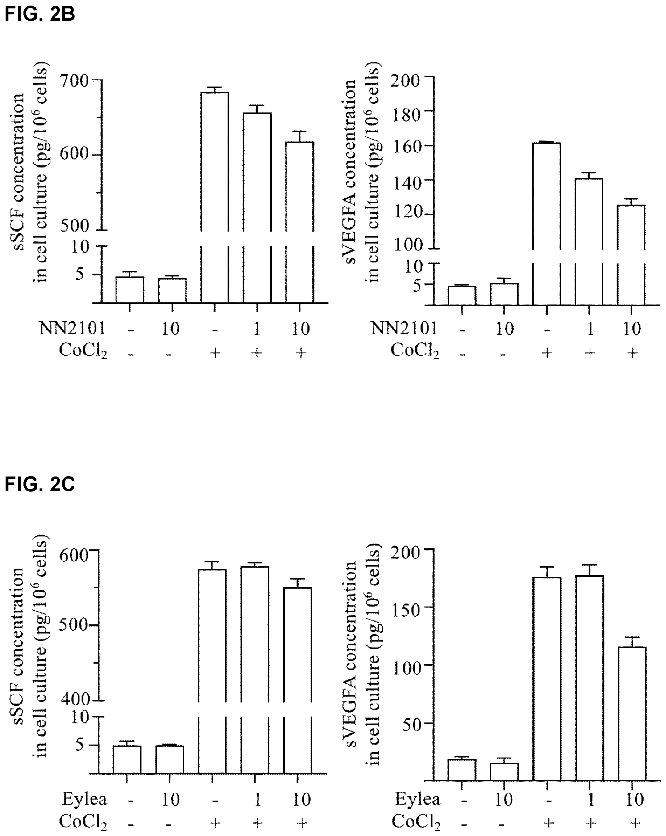

HRMECs (Cell Systems, Kirkland, WA, USA) were cultured overnight in EBM (Lonza, Walkersville, MD, USA) containing 0.5% FBS and then treated as follows. The HRMECs were pretreated with 10 µg/mL NN2101 or 10 µg/mL Eylea (Bayer Korea Ltd., Seoul, Korea) for 6 hours and then treated for 2 hours with 100 µM CoCl₂ to induce hypoxic state. The protein expression level in the total cell lysate was analyzed by western blotting (FIG. 2a). The sSCF (FIGS. 2b and 2c, left) and sVEGF (FIGS. 2b and 2c, right) secreted into the culture supernatant were quantified by ELISA.

As a result, NN2101 inhibited the expression of HIF-1α in the CoCl₂-induced hypoxic state and, accordingly, the expression of SCF and VEGFA was also reduced significantly and the signal of pho-cKIT was blocked. In contrast, Eylea did not affect the level of HIF-1α.

### Example 4: NN2101 regulates expression of HIF-1α and phosphorylation of GSK-3β in hypoxic condition in presence of SCF.

It was also investigated in this example whether NN2101 inhibits the expression of HIF-1α in hypoxic state, as in Example 3. The activation of GSK-3β contributes to angiogenesis and the phosphorylation of GSK-3β is regulated by SCF/c-KIT signaling. Therefore, it was investigated whether the SCF/c-KIT signaling mechanism is activated by confirming the degree of GSK-3β phosphorylation.

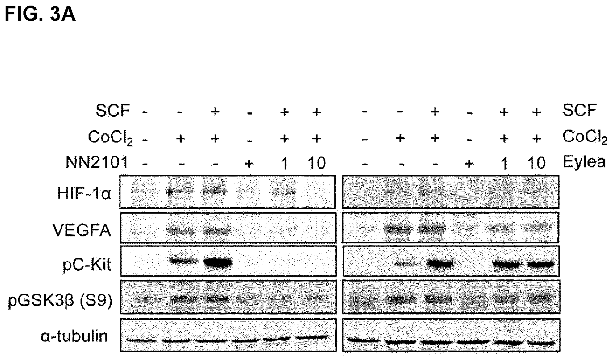

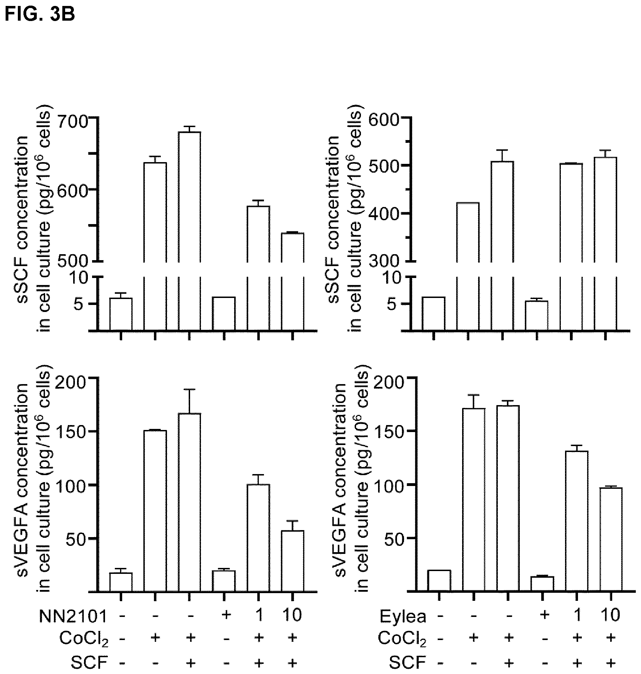

HRMECs were cultured overnight in EBM containing 0.5% FBS and then treated as follows. The HRMECs were pretreated with NN2101 (1 or 10 µg/mL) or Eylea (1 or 10 µg/mL) for 6 hours and then treated for 2 hours with 100 µM CoCl₂ and/or 100 ng/mL SCF. The protein expression level in the total cell lysate for 2 hours was analyzed by western blotting (FIG. 3a). The sSCF (FIG. 3b, top) and sVEGF (FIG. 3b, bottom) secreted into the culture supernatant were quantified by ELISA.

As a result, NN2101 reduced the expression of HIF-1α in hypoxic state in the presence of SCF in a concentration-dependent manner and, accordingly, the expression of SCF and VEGFA was also reduced significantly. In addition, the activation of cKIT was blocked due to phosphorylation (decreased expression of phospho-cKIT), and the phosphorylation of GSK-3β was also inhibited.

### Example 5: NN2101 quickly inhibits the expression of HIF-1α protein.

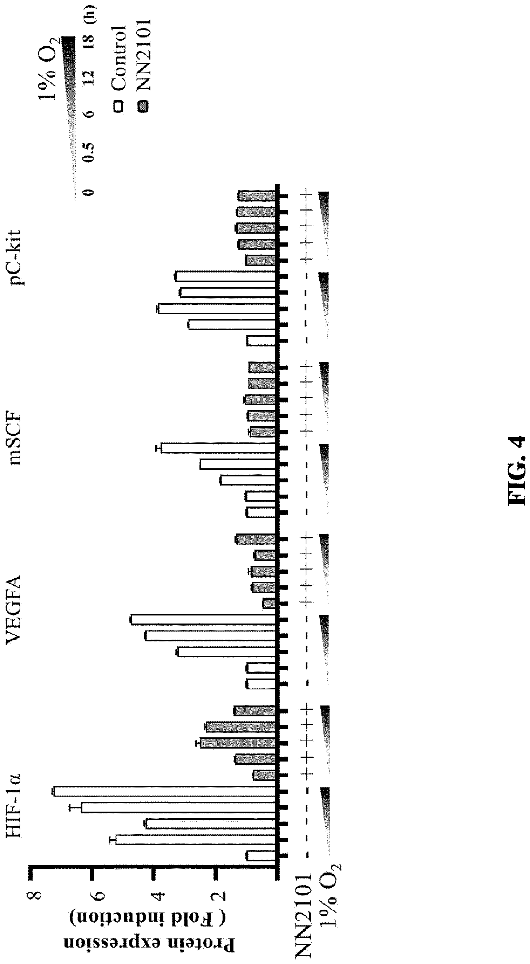

HRMECs were cultured overnight in EBM containing 0.5% FBS and then treated as follows. The HRMECs were pretreated with 10 µg/mL NN2101 for 6 hours on a plate and then exposed for 0.5, 6, 12 or 18 hours to 1% O₂ in order to induce hypoxic state. The protein expression level in the total cell lysate was analyzed by western blotting (FIG. 4).

As a result, the expression of HIF-1α, VEGFA, mSCF, pho-cKIT and proteins was reduced significantly by the treatment with NN2101 in the hypoxic state.

### Example 6: NN2101 down-regulates mRNA expression of HIF-1α target gene in HRMECs.

It is known that the proteins such as VEGF, GLUT1, GLUT3, etc. are expressed together when HIF-1α is expressed in the hypoxic state. In this example, it was investigated whether NN2101 inhibits the expression of HIF-1α by investigating the mRNA expression level of the target proteins.

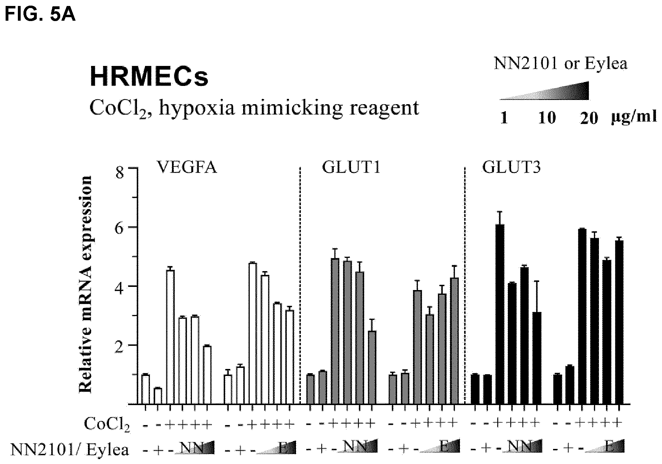

HRMECs were cultured overnight in EBM containing 0.5% FBS and then treated as follows. The HRMECs were pretreated with NN2101 or Eylea (1, 10 or 20 µg/mL) for 6 hours and then treated for 2 hours with 100 µM CoCl₂ in order to induce hypoxic state (FIG. 5a).

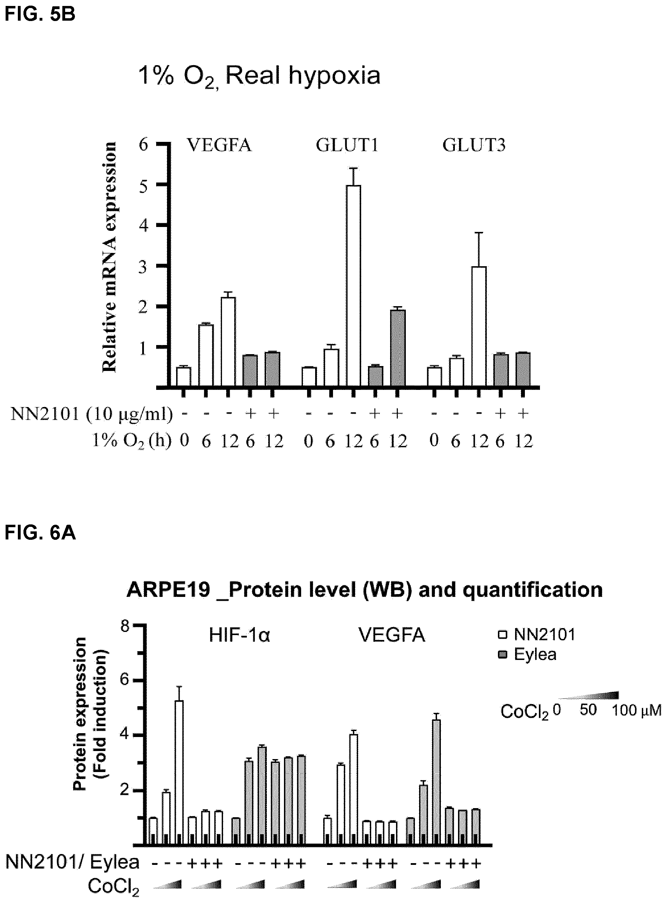

Separately, in order to investigate the expression of HIF-1α at actual hypoxic concentration, HRMECs were pretreated for 6 hours with 10 µg/mL NN2101 and then exposed for 6 or 12 hours to 1% O₂ (FIG. 5b). The mRNA expression level was analyzed by real-time PCR.

As a result, the treatment of the HRMECs with NN2101 in the hypoxic state down-regulated the mRNA level of VEGFA, GLUT1 or GLUT3, which are the target proteins of HIF-1α expression. In contrast, although Eylea slightly decreased the mRNA level of VEGFA, it hardly regulated the expression of GLUT1 or GLUT3.

### Example 7: NN2101 significantly inhibits expression of HIF-1α target gene in retinal pigment epithelial (RPE) cells.

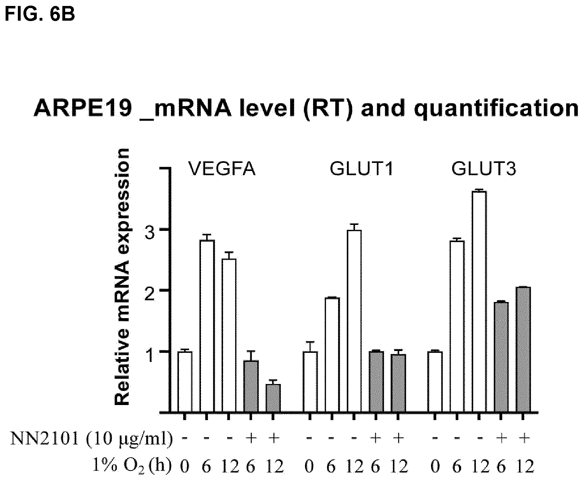

ARPE-19 cells (American Type Culture Collection, Manassas, VA, USA) were cultured overnight in DMEM-F12 containing 1% FBS. The cells were pretreated with 10 µg/mL NN2101 or 10 µg/mL Eylea for 6 hours and then hypoxia or hypoxic state was induced by treating with 50 or 100 µM CoCl₂ (FIG. 6a) or exposing for 0.5, 6 or 12 hours to 1% O₂ (FIG. 6b).

As a result, the protein level of the HIF-1α target gene (FIG. 6a) and the mRNA level of the HIF-1α target gene (FIG. 6b) were regulated by the treatment of the RPE cells with NN2101 in the hypoxic state.

### Example 8: NN2101 inhibits expression of HIF-1α, VEGFA, etc. in primary retinal cells in hypoxic state (1% O₂) as compared to Eylea.

Four retinal cells; HRMECs (human retinal microvascular endothelial cells), ARPE19 cells (spontaneously arising retinal pigment epithelial cells), MIO-ML cells (Moorfields /Institute of Ophthalmology-Mueller 1; spontaneously immortalized human Mueller glia cells) and Y79 cells (photoreceptor cells) were cultured as follows. The HRMECs were incubated overnight in EBM. The ARPE19 cells were incubated overnight in DMEM/F12. The MIO-ML cells (Mueller glial) were incubated overnight in DMEM. The MIO-ML cells were provided by Professor G. Astrid Limb (Institute of Ophthalmology, University College London, London, UK). The Y79 cells (American Type Culture Collection, Manassas, VA, USA) were incubated overnight in RPMI1640.

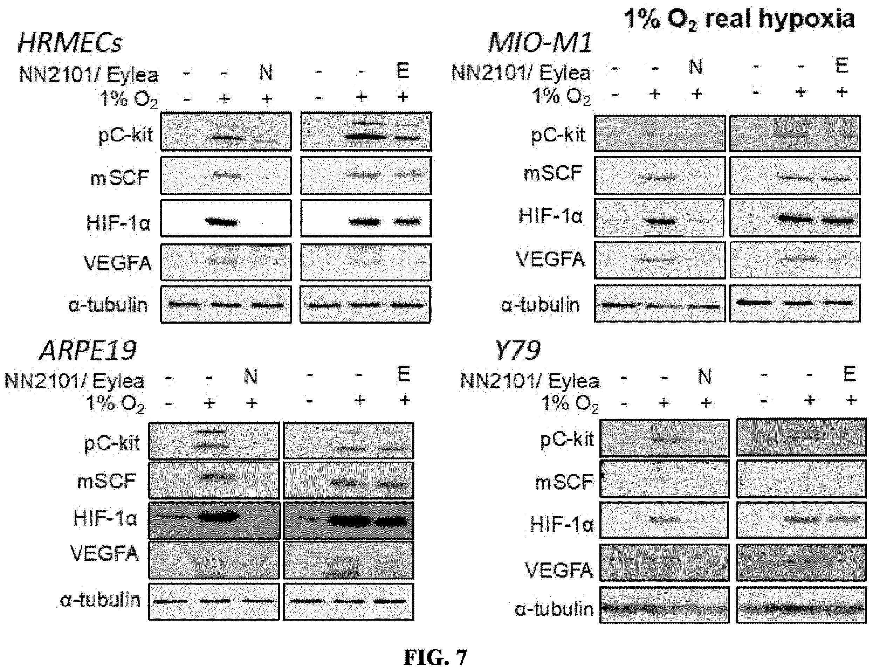

The four cells were treated indicated combinations. The cells were pretreated with the NN2101 antibody (10 µg/mL) or Eylea (10 µg/mL) for 6 hours and then exposed to 1% O₂ for 18 hours. The protein expression level in the total cell lysate was analyzed by western blotting (FIG. 7).

As a result, the expression of HIF-1α in the primary retinal cells was regulated by the treatment with NN2101 and, accordingly, the expression of SCF and VEGFA was decreased significantly and the signal of pho-cKIT was blocked. In contrast, Eylea did not affect the level of HIF-1α, mSCF, VEGFA and phospho-cKIT.

### Example 9: NN2101 facilitates degradation of HIF-1α via ubiquitin-proteasome degradation pathway.

### <Example 9-1>

HIF-1α is hydroxylated by prolyl hydroxylase (PHD) under normal oxygen conditions and then degraded by proteasomes through pVHL-mediated ubiquitination. In hypoxic state, the hydroxylation is inhibited and HIF-1α which enters the nucleus dimerizes with HIF-1β and promotes the transcriptional expression of target genes that facilitate neovascularization, etc. In this example, it was investigated whether NN2101 is involved in protein expression via the ubiquitin-proteasome degradation pathway of HIF-1α. To this end, MG132 (S2619, Selleckchem, Houston, USA) which is a representative proteasomal inhibitor and cyclohexamide (CHx) (S7418, Selleckchem, Houston, USA) which inhibits de novo synthesis of proteins from mRNAs were used.

HRMECs were incubated overnight in EBM containing 0.5% FBS. The cells were pretreated with NN2101 (10 µg/mL) for 6 hours and then treated with 50 µM CoCl₂ for 12 hours in order to induce hypoxic state. After the treatment, 10 µmol/L MG132 or CHx was added for the indicated period. The total HIF-1α expression level in the cells was analyzed by western blot. The density of each protein band was determined using an image analysis system and normalized to the density of the corresponding α-tubulin.

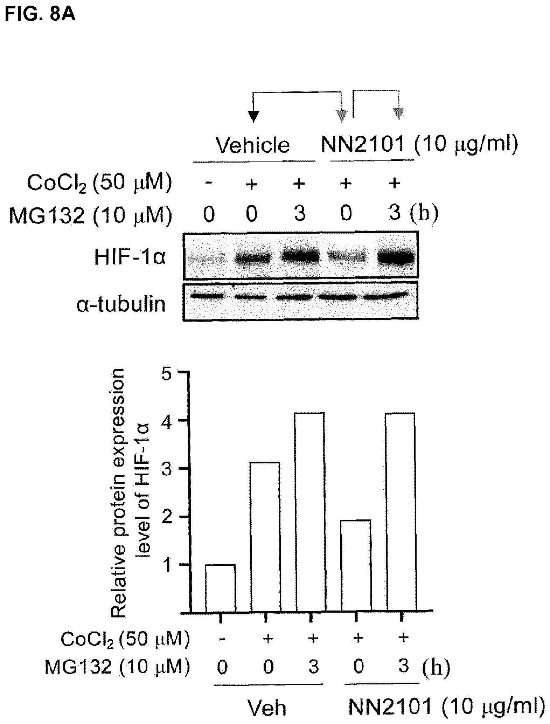

As a result, the level of HIF-1α increased by the induction of hypoxic state with CoCl₂ (FIG. 8a, second lane) was decreased by the treatment with NN2101 (FIG. 8a, fourth lane). The decreased HIF-1α was recovered to the level of a vehicle-treated group by the treatment with the proteasomal inhibitor MG132 (FIG. 8a, third lane vs fifth lane). The restoration of the HIF-1α level by the treatment with MG132 to a level comparable to that of the treatment with NN2101 suggests that NN2101 is involved in the ubiquitin-proteasome pathway of HIF-1α.

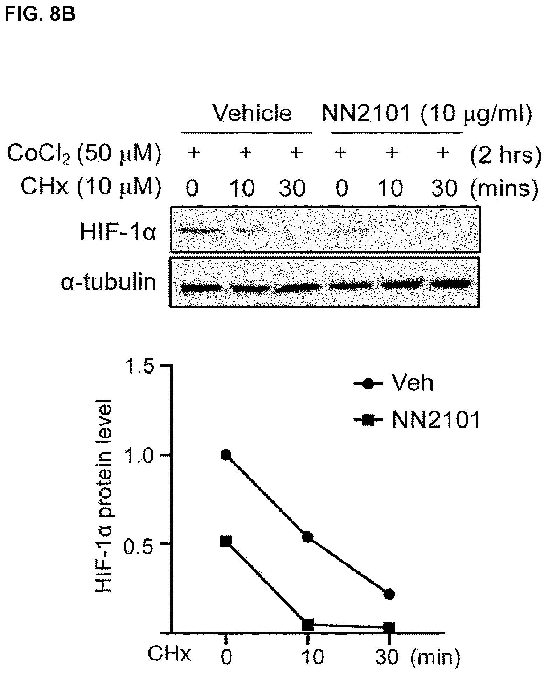

In addition, the half-life of HIF-1α was measured by treating with CHx which inhibits protein synthesis. It was confirmed that the slope of the half-life of the NN2101-treated group (FIG. 8b bottom; squares) was steeper than that of the control group (vehicle) (FIG. 8b bottom; circles). This suggests that NN2101 accelerates the protein degradation rate of HIF-1α (FIG. 8b).

### <Example 9-2>

In this example, the relationship between NN2101 and HIF-1α degradation was investigated by overexpressing PHD in HUVEC cells (Lonza, Walkersville, MD, USA).

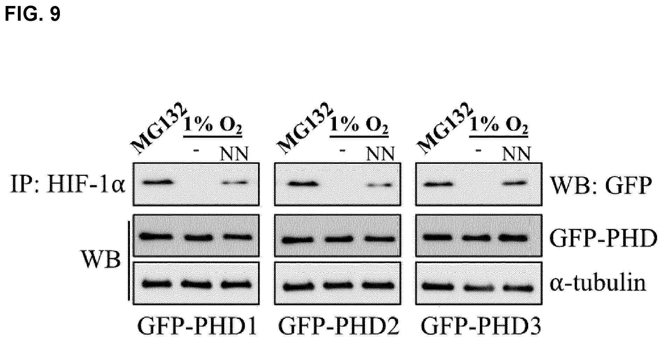

For this, HUVEC cells were overexpressed using expression vectors encoding three isotypes of PHD to which green fluorescent protein (GFP) was attached, i.e., GFP-PHD1, GFP-PHD2 and GFP-PHD3. The transfected cells were incubated for 12 hours in hypoxic state or normal oxygen state by treating with 10 µg of NN2101. The cells were treated or untreated with 10 µmol/L MG132 for 3 hours before harvesting. HIF-1α was selectively immunoprecipitated (IP) from the total cell lysate and then HIF-1α bound to overexpressed PHD (HIF-1α-PHD) was analyzed by western blotting (FIG. 9).

As a result, the amount of HIF-1α-PHD was increased when the cells were treated only with the proteasomal inhibitor MG132 under normal oxygen condition (FIG. 9, first lane). HIF-1α-PHD was hardly detected in the hypoxic state (FIG. 9, second lane). In contrast, in the presence of NN2101, the amount of HIF-1α-PHD was increased in the hypoxic state (FIG. 9, third lane). This result also suggests that NN2101 is involved in the ubiquitin-proteasomal degradation of HIF-1α in the hypoxic state.

### Example 10: NN2101 reduces up-regulation of pro-angiogenesis/inflammation factors including HIF target gene in primary retinal cells in hypoxic state.

Four retinal cells, i.e., HRMECs (human retinal microvascular endothelial cells), ARPE19 cells (spontaneously arising retinal pigment epithelial cells), MIO-ML cells (Moorfields /Institute of Ophthalmology-Mueller 1; spontaneously immortalized human Mueller glia cells) and Y79 cells (photoreceptor cells), were cultured and treated as follows.

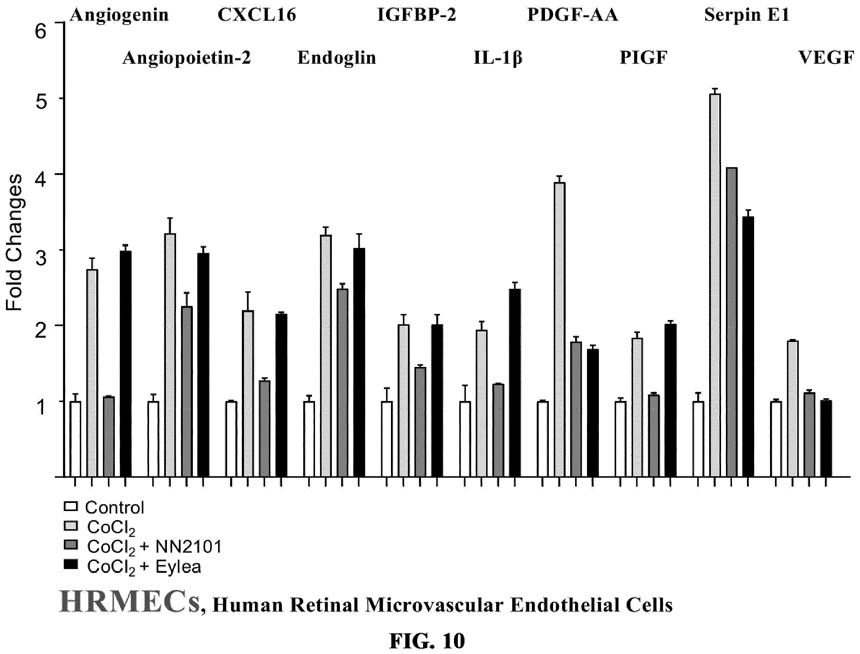

HRMECs were cultured overnight in EBM containing 0.5% FBS. The cells were pretreated for 6 hours with NN2101 antibody or Eylea (10 µg/mL) and then treated for 18 hours with 100 µM CoCl₂. The culture supernatant was analyzed and quantified with a proteome profiler. As a result, it was confirmed that NN2101 regulates neovascularization/inflammatory factors increased in the HRMECs in hypoxic state (FIG. 10).

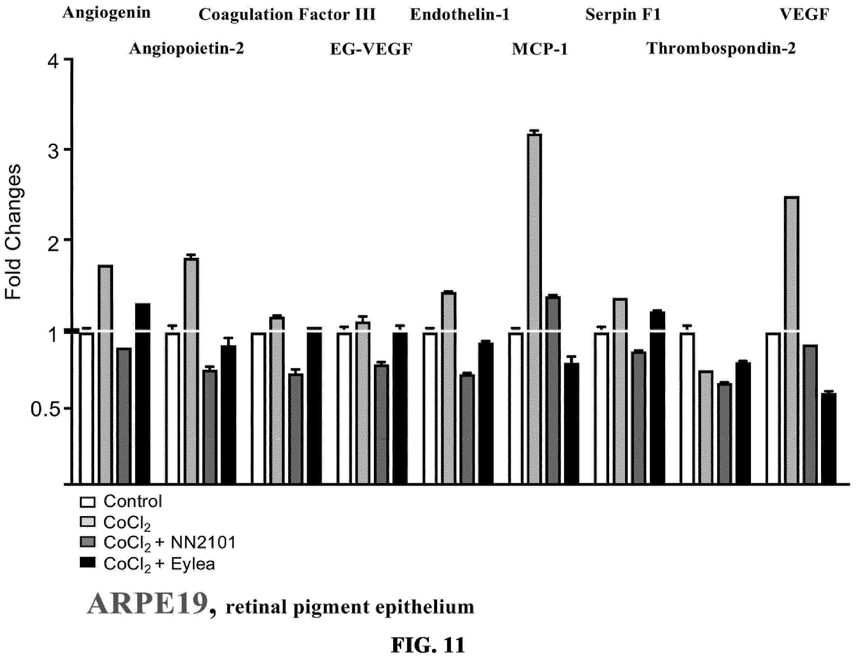

ARPE19 cells were cultured overnight in DMEM/F12 containing 0.5% FBS. The cells were pretreated for 6 hours with NN2101 mAb or Eylea (10 µg/mL) and then treated for 18 hours with 100 µM CoCl₂. The culture supernatant was analyzed and quantified with a proteome profiler. As a result, it was confirmed that NN2101 regulates neovascularization/inflammatory factors increased in the RPE cells in hypoxic state (FIG. 11).

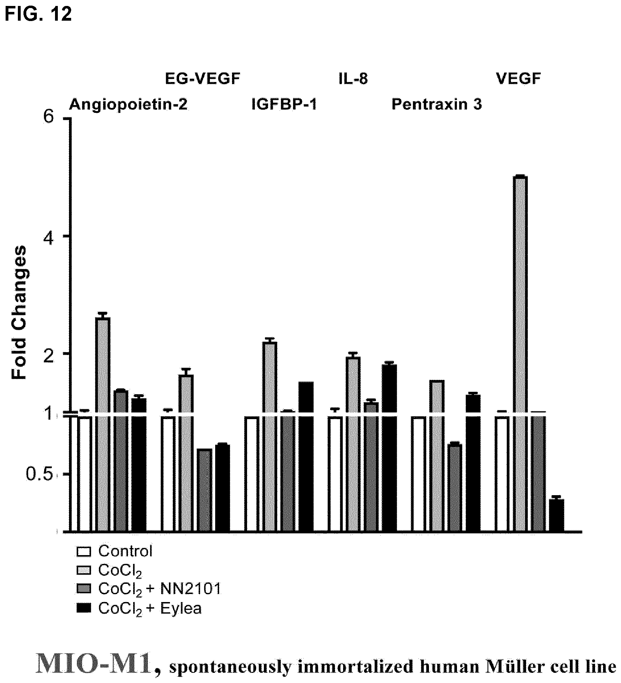

MIO-ML cells were cultured overnight in DMEM containing 0.5% FBS. The cells were pretreated for 6 hours with NN2101 mAb or Eylea (10 µg/mL) and then treated for 18 hours with 100 µM CoCl₂. The culture supernatant was analyzed and quantified with a proteome profiler. As a result, it was confirmed that NN2101 regulates neovascularization/inflammatory factors increased in the MIO-ML cells in hypoxic state (FIG. 12).

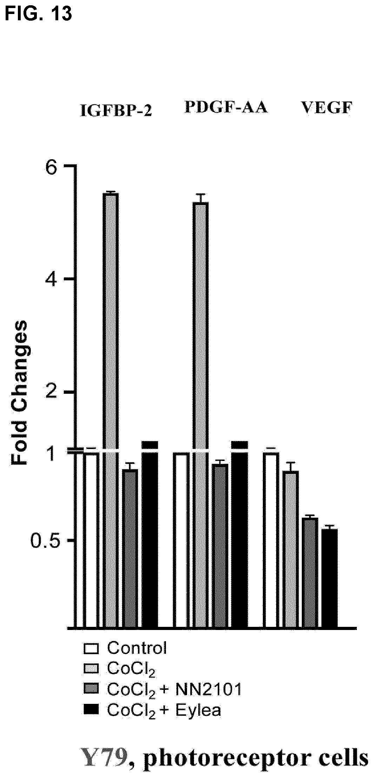

Y79 cells were cultured overnight in RPMI1640 containing 0.5% FBS. The cells were pretreated for 6 hours with NN2101 mAb or Eylea (10 µg/mL) and then treated for 18 hours with 100 µM CoCl₂. The culture supernatant was analyzed and quantified with a proteome profiler. As a result, it was confirmed that NN2101 regulates neovascularization/inflammatory factors increased in the Y79 cells in hypoxic state (FIG. 13).

In conclusion, it was confirmed that NN2101 regulates the neovascularization/inflammatory factors in different cells in hypoxic state (FIGS. 10-13). In particular, NN2101 reduced angiopoietin-2 and VEGF at the same time and regulated other angiogenic factors. This suggests that NN2101 will exhibit comparable or better effect as compared to faricimab which targets angiopoietin-2 and VEGF at the same time.

Although the specific exemplary embodiments of the present disclosure have been described, those having ordinary knowledge in the art will be able to modify and change the present disclosure variously through addition, change, deletion, addition, etc. of elements without departing from the idea of the present disclosure.

## Claims

1. A method for preventing or treating an eye disease that is refractory or non-responsive to existing eye disease drugs by inhibiting the expression or activity of HIF-1α, comprising:
a step of administering a therapeutically effective amount of an antibody comprising the following CDR sequences or an antigen-binding fragment thereof, a nucleic acid molecule encoding the antibody or antigen-binding fragment thereof, or a vector containing the nucleic acid molecule to a subject in need thereof:
(i) a light-chain CDR1 of the amino acid sequence represented by SEQ ID NO 1;
(ii) a light-chain CDR2 of the amino acid sequence represented by SEQ ID NO 2;
(iii) a light-chain CDR3 of the amino acid sequence represented by SEQ ID NO 3;
(iv) a heavy-chain CDR1 of the amino acid sequence represented by SEQ ID NO 4;
(v) a heavy-chain CDR2 of the amino acid sequence represented by SEQ ID NO 5; and
(vi) a heavy-chain CDR3 of the amino acid sequence represented by SEQ ID NO 6.

2. The method for preventing or treating an eye disease according to claim 1, wherein the eye disease is a retinal or macular disease.

3. The method for preventing or treating an eye disease according to claim 2, wherein the retinal or macular disease is one or more disease selected from the group consisting of choroidal angiogenesis, maculopathy, retinopathy, retinal cell degeneration, retinal vascular occlusion, retinal detachment and glaucoma.

4. The method for preventing or treating an eye disease according to claim 3, wherein the retinal or macular disease is one or more disease selected from the group consisting of diabetic retinopathy, diabetic macular edema, wet age-related macular degeneration, retinal vein occlusion, retinopathy of prematurity and glaucoma.

5. The method for preventing or treating an eye disease according to claim 1, wherein the existing eye disease drug is a VEGF inhibitor.

6. The method for preventing or treating an eye disease according to claim 1, wherein the existing eye disease drug is selected from the group consisting of aflibercept, ranibizumab, Susvimo^{®}, bevacizumab, brolucizumab, pegaptanib, faricimab and KSI-301.

7. A method for inhibiting the expression or activity of HIF-1α, comprising a step of administering a therapeutically effective amount of an antibody comprising the following CDR sequences or an antigen-binding fragment thereof, a nucleic acid molecule encoding the antibody or antigen-binding fragment thereof, or a vector containing the nucleic acid molecule to a subject in need thereof:
(i) a light-chain CDR1 of the amino acid sequence represented by SEQ ID NO 1;
(ii) a light-chain CDR2 of the amino acid sequence represented by SEQ ID NO 2;
(iii) a light-chain CDR3 of the amino acid sequence represented by SEQ ID NO 3;
(iv) a heavy-chain CDR1 of the amino acid sequence represented by SEQ ID NO 4;
(v) a heavy-chain CDR2 of the amino acid sequence represented by SEQ ID NO 5; and
(vi) a heavy-chain CDR3 of the amino acid sequence represented by SEQ ID NO 6.

8. The method for inhibiting the expression or activity of HIF-1α according to claim 7, wherein the antibody or antigen-binding fragment thereof comprises a light-chain variable region of the amino acid sequence represented by SEQ ID NO 7 and a heavy-chain variable region of the amino acid sequence represented by SEQ ID NO 8.

9. The method for inhibiting the expression or activity of HIF-1α according to claim 7, wherein the inhibition of the expression or activity of HIF-1α is accomplished in the choroid or retina.

10. The method for inhibiting the expression or activity of HIF-1α according to claim 9, wherein the inhibition of the expression or activity of HIF-1α is accomplished in cells selected from the group consisting of optic neurons, retinal Mueller cells, choroidal cells, retinal microvascular endothelial cells (RMECs), retinal pigment epithelial (RPE) cells and photoreceptor cells.

11. The method for inhibiting the expression or activity of HIF-1α according to claim 7, wherein the inhibition of the expression of HIF-1α is the inhibition of the expression of the HIF-1α protein.

12. The method for inhibiting the expression or activity of HIF-1α according to claim 7, wherein the inhibition of the expression or activity of HIF-1α is achieved by facilitating the degradation of the HIF-1α protein.

13. The method for inhibiting the expression or activity of HIF-1α according to claim 7, wherein the inhibition of the activity of HIF-1α is achieved by inhibiting the activity of HIF-1α through SCF/C-Kit-induced phosphorylation.

14. The method for inhibiting the expression or activity of HIF-1α according to claim 7, wherein the antibody or antigen-binding fragment thereof further regulates angiogenic factors and/or inflammatory factors.

15. The method for inhibiting the expression or activity of HIF-1α according to claim 14, wherein the angiogenic factors and/or inflammatory factors are one or more factors selected from the group consisting of angiogenin, angiopoietin-2, CXCL16, endoglin, coagulation factor III, endotherin-1, MCP-1, EG-VEGF, VEGF, IGFBP-1, IGFBP-2, IL-113, PDGF-AA, PIGF, serpin E, serpin F1, thrombospondin-2, ANGPT2, pentraxin 3, IL-8, FGF2 and FGF5.

16. The method for inhibiting the expression or activity of HIF-1α according to claim 14, wherein the antibody or antigen-binding fragment thereof reduces the up-regulation of the expression of angiogenic factors and/or inflammatory factors.

17. The method for inhibiting the expression or activity of HIF-1α according to claim 7, wherein the antibody or antigen-binding fragment thereof reduces any one selected from the group consisting of the proliferation, migration and tube formation of microvascular cells.

18. A pharmaceutical composition for preventing or treating an eye disease that is refractory or non-responsive to existing eye disease drugs by inhibiting the expression or activity of HIF-1α, comprising an antibody comprising the following CDR sequences or an antigen-binding fragment thereof, a nucleic acid molecule encoding the antibody or antigen-binding fragment thereof, or a vector containing the nucleic acid molecule as an active ingredient:
(i) a light-chain CDR1 of the amino acid sequence represented by SEQ ID NO 1;
(ii) a light-chain CDR2 of the amino acid sequence represented by SEQ ID NO 2;
(iii) a light-chain CDR3 of the amino acid sequence represented by SEQ ID NO 3;
(iv) a heavy-chain CDR1 of the amino acid sequence represented by SEQ ID NO 4;
(v) a heavy-chain CDR2 of the amino acid sequence represented by SEQ ID NO 5; and
(vi) a heavy-chain CDR3 of the amino acid sequence represented by SEQ ID NO 6.

19. The use of an antibody comprising the following CDR sequences or an antigen-binding fragment thereof, a nucleic acid molecule encoding the antibody or antigen-binding fragment thereof, or a vector containing the nucleic acid molecule, for treatment of an eye disease that is refractory or non-responsive to existing eye disease drugs by inhibiting the expression or activity of HIF-1α:
(i) a light-chain CDR1 of the amino acid sequence represented by SEQ ID NO 1;
(ii) a light-chain CDR2 of the amino acid sequence represented by SEQ ID NO 2;
(iii) a light-chain CDR3 of the amino acid sequence represented by SEQ ID NO 3;
(iv) a heavy-chain CDR1 of the amino acid sequence represented by SEQ ID NO 4;
(v) a heavy-chain CDR2 of the amino acid sequence represented by SEQ ID NO 5; and
(vi) a heavy-chain CDR3 of the amino acid sequence represented by SEQ ID NO 6.
